# EUROPEAN PATENT SPECIFICATION

(11) **EP 2 117 334 B1**
(45) Date of publication and mention of the grant of the patent: **14.11.2018**
(21) Application number: 08705462.3
(22) Date of filing: 03.01.2008
(51) Int. Cl.: A23K 20/158, A23K 50/00, A23K 50/10, A23K 50/75, A23K 50/30, A23K 50/80, A23L 27/60, A23L 33/12, A23C 9/152, A23C 11/10, A23C 19/05, A23D 7/00, A23D 9/00, A23J 3/16

(54) **FOOD COMPOSITIONS INCORPORATING ADDITIONAL LONG CHAIN FATTY ACIDS**
NAHRUNGSMITTELZUSAMMENSETZUNGEN MIT ZUSÄTZLICHEN LANGKETTIGEN FETTSÄUREN
COMPOSITIONS ALIMENTAIRES CONTENANT DES ACIDES GRAS À LONGUE CHAÎNE SUPPLÉMENTAIRES

(30) Priority: 03.01.2007 US 878300 P
(43) Date of publication of application: 18.11.2009
(73) Proprietor: Monsanto Technology, LLC, St. Louis, MO 63167 (US)
(72) Inventor: WILKES, Richard, S., St. Louis, MO 63167 (US)
(74) Representative: dompatent von Kreisler Selting Werner - Partnerschaft von Patent- und Rechtsanwälten mbB
(86) International application number: PCT/US2008/000051
(87) International publication number: WO 2008/085840

(56) References cited:
- EP-A1- 0 936 266
- WO-A1-2005/021761
- WO-A1-2007/056823
- WO-A2-2005/083093
- US-A1- 2004 172 682
- US-A1- 2004 172 682
- US-A1- 2006 111 578
- US-A1- 2006 111 578
- ANONYMOUS: "Monsanto launches Visitive low-linolenic soybeans", INFORM, AOCS, AMERICAN OIL CHEMISTS SOCIETY, CHAMPAIGN, IL, US, vol. 15, no. 11, 1 November 1994 (1994-11-01), page 752, XP008111134, ISSN: 0897-8026
- ANONYMOUS: 'Monsanto launches Visitive low-linolenic soybeans' INFORM vol. 15, no. 11, page 752, XP008111134

## Description

### FIELD OF THE INVENTION

The present invention relates to the utilization of transgenically derived stearidonic acid in the development of functional food products. More specifically it relates to an improvement in both the nutritional quality and shelf-life of food products through the use of transgenic plant-derived stearidonic acid.

### BACKGROUND OF THE INVENTION

Disclosed herein is a method for improving foodstuffs through the utilization of novel partially transgenic plant-derived long-chain polyunsaturated fatty acid compositions ("LC-PUFA"), in particular those with the positive attributes of Omega-3 fatty acids and enhanced stability through the reduction of linolenic acid. Specifically, the inventor provides techniques and methods for the utilization of plant-derived LC-PUFA in foodstuffs that improves nutritional quality when combined with oil from conventionally improved breeds of oil-producing plants. In the past dietary fats have been thought of as valueless or even harmful dietary components. Many studies have made a physiological link between dietary fats and obesity and other pathologies such as atherosclerosis. Given this perception of low nutritional value, consumption of fats has been discouraged by many in the medical establishment.

However, recent studies have determined that despite their relatively simple biological structures there are some types of fats that appear to improve body function in some ways and that may, in fact, be essential to certain physiological processes. The wider class of fat molecules includes fatty acids, isoprenols, steroids, other lipids and oil-soluble vitamins. Among these are the fatty acids. The fatty acids are carboxylic acids, which have from 2 to 26 carbons in their "backbone," with none, or various numbers of unsaturations in their carbohydrate structure. They generally have dissociation constants (pKa) of about 4.5 indicating that in normal body conditions (physiological pH of 7.4) the vast majority will be in a dissociated form.

With the improvement in nutritional stature for fats and in particular fatty acids, many in the food industry have begun to focus on fatty acids and lipid technology as a new focus for food production. This focus has been particularly intense for the production and incorporation of Omega-3 fatty acids into the diet. Omega-3 fatty acids are long-chain polyunsaturated fatty acids (18-22 carbon atoms in chain length) with the first of the double bonds ("unsaturations") beginning with the third carbon atom. They are called "polyunsaturated" because their molecules have two or more double bonds "unsaturations" in their carbohydrate chain. They are termed "long-chain" fatty acids since their carbon backbone has at least 18 carbon atoms. The LC-PUFA family of oils for food compositions includes: alpha linolenic acid ("ALA"), stearidonic acid ("SDA"), gamma linolenic acid ("GLA"), linoleic acid ("LA"). ALA is the "base" omega-3 fatty acid, from which SDA is made in the body through a series of enzymatic reactions, but can be reduced to provide a healthier oil composition. This synthesis processes from ALA are called "elongation" (the molecule becomes longer by incorporating new carbon atoms) and "desaturation" (new double bonds are created), respectively. In nature, ALA is primarily found in certain plant seeds (e.g., flax).

In addition to difficulties with simply securing an appropriate supply of LC-PUFA's for societal consumption often the costs to process LC-PUFA's into food products is restrictive. These Omega-3 fatty acids, and some of the other LC-PUFA's can be quickly oxidized leading to undesirable odors and flavors. To reduce the rate of oxidation food processors must therefore either distribute the oil in a frozen condition or encapsulate the desirable fatty acids, each greatly increasing the cost of processing and consequent cost to the consumer. Despite this increased expense - food companies are interested in supplying Omega-3's and generally healthier food oils because they believe that health conscious consumers may be willing to pay a small premium for an improved diet if a reliable supply can be developed.

Along with the movement of food companies to develop essential fats and oils as an important component in a healthy diet, governments have begun developing regulations pushing for the adoption of LC-PUFA's in the diet. The difficulty in supplying these needs has been the inability to develop a large enough supply of Omega-3 oil to align with growing marketplace demand. These limitations on supply, stability and sourcing greatly increase cost and correspondingly limit the availability of dietary Omega-3's. Accordingly, a need exists to provide a large-scale stable supply of Omega-3's to include in food and feed formulations in a commercially acceptable way.

In addition, soybean oil represents two-thirds of all food oil consumed in the United States. Food companies have used soybean oil because it is plentiful and relatively low cost. Soybean oil is typically low in harmful saturated fat and has a taste and texture desired by consumers. Currently, soybean oil accounts for roughly 80%, or 18.0 billion pounds, of the oil consumed in the US and is the most widely used oil in food production. However, to meet market expectations for shelf life, hydrogen must be added to soybean oil to increase its shelf-life and stability for use in processed foods such as fried foods, baked goods and snack products. This hydrogenation process creates trans fats.

Unfortunately, trans-fats have been linked to heart disease due to the findings that they have a negative impact on human cholesterol profiles. With this in mind the United States FDA has required food labels to include a trans fat content as from January 1, 2006. This has created a substantial demand for supplies of dietary oils that have lower levels of trans fats.
Accordingly, there is a market demand for a composition with lower trans fats with a profile that also includes other identifiable health benefits, such as Omega-3 fatty acids to meet federal guidelines and the demands of consumers for healthier food.
US7741500 discloses a process for refining, bleaching, and deodorizing oil under conditions that minimize oxidation and isomerization of the unsaturated fatty acids within the oil. The process can be used with seeds of transgenic plants, such as canola and soy, to give oils enriched in stearidonic acid. These oil compositions have advantageous stability characteristics and can be useful for nutritional, pharmaceutical, industrial, and other purposes.
WO 2005/021761 describes genetic modification of soy plants to produce seeds and soy oils having increased stearidonic acid concentration and further discloses a method of producing soybean seed with a modified fatty acid profile by expression of desaturase enzymes in transgenic soybean plants and methods of increasing the nutritional value of edible products for human or animal consumption utilizing the soy oil.
WO 2005/083093 describes a process for the production of arachidonic acid, eicosapentaenoic acid or docosahexaenoic acid and a process for the production of triglycerides with an elevated content of unsaturated fatty acids, in particular arachidonic acid, eicosapentaenoic acid and/or docosahexaenoic acid, in transgenic plants, advantageously in the seed of the transgenic plant. It relates to the generation of a transgenic plant with an elevated content of polyunsaturated fatty acids, in particular arachidonic acid, eicosapentaenoic acid and/or docosahexaenoic acid, as the result of the expression of the elongases and desaturases used in the described process.
WO 2007/056823 describes feedstuffs for use in aquaculture. The fish or crustaceans are fed a feedstuff comprising lipid, wherein the fatty acid of the lipid comprises at least 5.5 wt.% stearidonic acid (SDA). The SDA may be provided in the form of a transgenic organism, or extract or portion thereof. Alternatively, the SDA may be provided in the form of a plant oil from a non-transgenic organism.

The current invention provides an invention that answers both of the needs described above. It offers an alternative to fish or microbe supplied Omega-3 fatty acids and provides food/feed product comprising a soybean oil that has lower linolenic acid content, improving its taste profile and enhancing shelf-life without the production of trans fats through hydrogenation. The technology relied upon is both conventional plant breeding technology, oil processing technology and transgenically developed plants. The plant species that are specifically included within the group of those that could supply demand are: soybeans, corn, and canola, but also may include other plants as needed. Once produced the LC-PUFA's of the food/feed products of the invention can be used to improve the health characteristics of a great variety of food products. This production can also be scaled-up as needed to both reduce the need to harvest wild fish stocks and to provide essential fatty acid components for aquaculture operations, each easing pressure on global fisheries.

Surprisingly, the inventor has found that the concentration of LC-PUFA's from transgenic plant sources disclosed herein require a lower concentration in a given food or beverage product to be physiologically significant, these ranges are well within acceptable volume parameters for typical food products and can be used for a wider variety of foodstuffs.

### SUMMARY OF THE INVENTION

The present invention encompasses the use of a transgenic plant oil comprising stearidonic acid and a soybean oil comprising less than 3 % linolenic acid based on the fatty acid composition of the soybean oil to form a mixture for increasing the shelf-life of a food product. Several food products made from the oil mixture have been produced and found to have similar taste and sensory properties compared to products made from conventional oils, such as soybean oil.

The plant-derived oil mixture has substantially improved shelf-life characteristics relative to other Omega-3 containing products.

Nutritional studies have shown that, compared to alpha-linolenic acid, SDA is about 5 times more efficiently converted *in vivo* to EPA. Accordingly, plant-derived oil mixtures can be utilized as a neutraceutical supplement or dietary additive for certain pathological conditions with a lengthened shelf life due to a lower oxidation rate.

A plant-derived oil mixture can provide an oil reduced in trans-fats that can synergistically improve the health profile of the delivered oil by also delivering the health benefits of Omega-3 oil.

Specifically, acceptable food products can be made with stearidonic acid, increasing their shelf-life beyond that of competitive oils.

Other features and advantages of this invention will become apparent in the following detailed description of preferred embodiments of this invention, taken with reference to the accompanying figures.

### BRIEF DES CRIPTION OF THE DRAWINGS

*Figures that reference "SDA + Vistive™" also comprise the LC-PUFA oil described herein.
Fig. 1 Shows The Biosynthetic Pathway Of PUFA Metabolism.
Fig. 2 Shows Time Point Testing For Sensory Information For Italian Dressing A-E.
Fig. 3 Shows Time Point Testing For Sensory Information For Ranch Dressing A-E.
Fig. 4 Shows Time Point Testing For Sensory Information For Mayonnaise A-D.

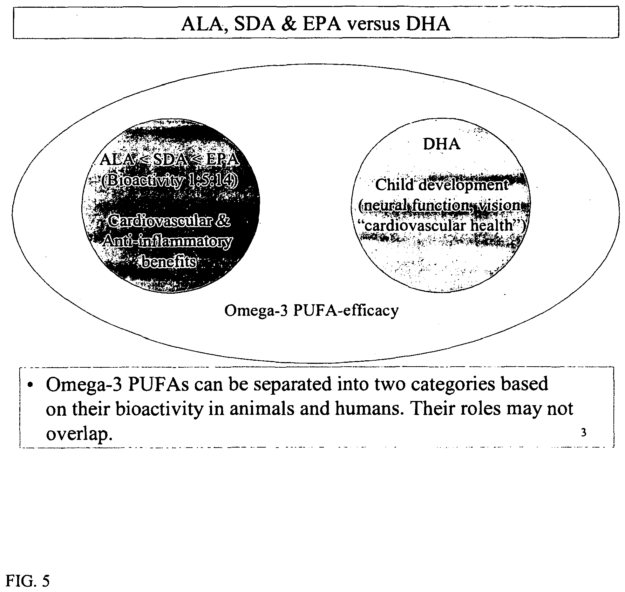

Fig. 5 Shows A Graphic Representing The Relative Bioactivity Of Omega-3 Fatty Acids.
Fig. 6 Shows A Process Flow Diagram For The Production Of Soymilk.
Fig. 7 Shows A Process Flow Diagram For The Production Of Vanilla Soymilk.

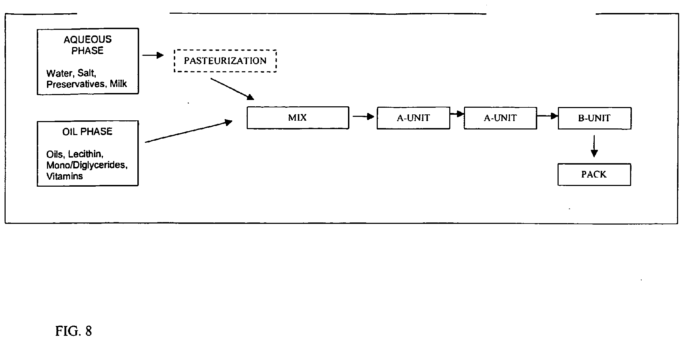

Fig. 8 Shows A Process Flow Diagram For The Production Of Margarine.

### DESCRIPTION OF THE PREFERRED EMBODIMENT

The following abbreviations have designated meanings in the specification:

### Abbreviation Key:

- AA: Arachidonic Acid
- ALA: α- Linolenic Acid
- DHA: Docosahexanoic Acid
- DNA: Deoxyribonucleic Acid
- EPA: Eicosapentanoic Acid
- GLA: γ- Linolenic Acid
- LA: Linoleic Acid
- mRNA: messenger Ribonucleic Acid
- PUFA: Poly-Unsaturated Fatty Acids
- SDA: Stearidonic Acid

### Explanation of Terms:

Expression - The process of the transcription of a gene to produce the corresponding mRNA and translation of this mRNA to produce the corresponding gene product (i.e., a peptide, polypeptide, or protein).

Food - Substances which are ingested by humans and contain nutrients which can be metabolized to produce energy.

Gene - Chromosomal DNA, plasmid DNA, cDNA, synthetic DNA, or other DNA that encodes a peptide, polypeptide, protein, or RNA molecule.

Host or Host Organism - Bacteria cells, fungi, animals and animal cells, plants and plant cells, or any plant parts or tissues including protoplasts, calli, roots, tubers, seeds, stems, leaves, seedlings, embryos, and pollen.

Mouthfeel - Means how the substance feels in a human mouth. With regard to taste test profiles this refers to the viscosity, texture and smoothness of the substance being tested.

Nutritional Food Bar - As used herein, the term "Nutritional Food Bar" means a food bar designed to promote health.

Transformation - refers to the introduction of nucleic acid into a recipient host.

Transgene - Any piece of a nucleic acid molecule that is inserted by artifice into a cell, or an ancestor thereof, and becomes part of the genome of the plant or animal which develops from that cell. Such a transgene may include a gene which is partly or entirely exogenous (i.e., foreign) to the transgenic plant or animal, or may represent a gene having identity to an endogenous gene of the plant or animal.

Transgenic - Any cell that includes a nucleic acid molecule that has been inserted by artifice into a cell, or an ancestor thereof, and becomes part of the genome of the plant or animal which develops from that cell.

### DETAILED DESCRIPTION

Disclosed herein is a system for an improved method of production of stearidonic acid and its incorporation into the diets of humans in an effort to improve human health. This production is through the utilization of transgenic plants engineered to produce LC-PUFA in high yield to allow commercial incorporation into food products. For the purposes of the current invention the acid and salt forms of fatty acids, for instance, butyric acid and butyrate, arachidonic acid and arachidonate, will be considered interchangeable chemical forms.

The oil composition of the food products disclosed herein provides for a lower linolenic acid profile than known soybean compositions while providing the benefits of Omega-3 derived stearidonic acid. The oil composition contains soybean oil that has less than 3% by weight linolenic acid based on the total weight of fatty acids, compared to 8% for traditional soybean oils. This results in a more stable soybean oil because with less linolenic acid the oil itself will oxidize more slowly resulting in superior shelf life. Also the flavor notes of linolenic acid are such that with a composition lower in this compound the oil will have a more palatable flavor profile. In addition soybeans with less linolenic acid require less or no partial hydrogenation. Therefore the production of undesirable trans fats in processed soybean oil can be reduced or eliminated and the corresponding oil will have a better cooking profile.

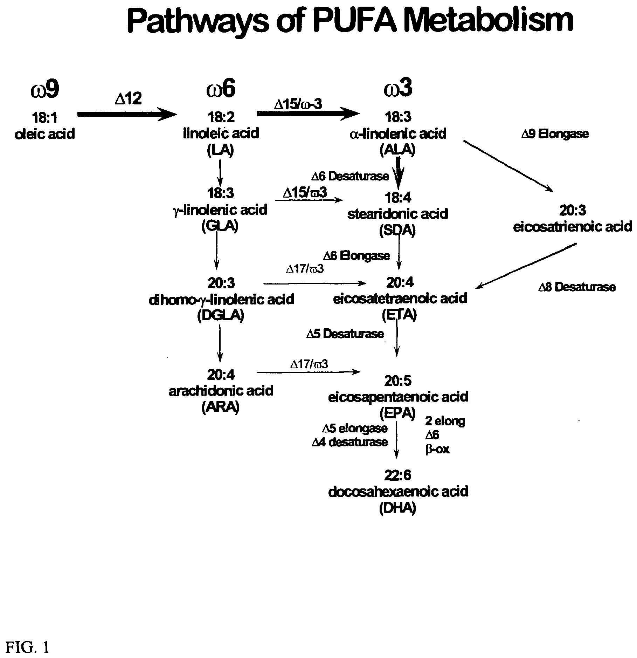

Turning to FIG. 1, all higher plants have the ability to synthesize the main 18 carbon PUFA's, LA and ALA, and in some cases SDA (C18:4n3, SDA), but few are able to further elongate and desaturate these to produce AA, EPA or DHA. Synthesis of EPA and/or DHA in higher plants therefore requires the introduction of several genes encoding all of the biosynthetic enzymes required to convert LA into AA, or ALA into EPA and DHA. Taking into account the importance of PUFAs in human health, the successful production of PUFAs (especially the n-3 class) in transgenic oilseeds can then provide a sustainable source of these essential fatty acids for dietary use. The "conventional" aerobic pathway which operates in most PUFA-synthesising eukaryotic organisms, starts with Δ6 desaturation of both LA and ALA to yield γ-linolenic (GLA, 18:3n6) and SDA.

### Establishing the Composition of Oils

Turning to Table 1a, it is important to provide a basis of what constitutes 'normal' ranges of oil composition vis-à-vis the oil compositions of the food/feed products of the current invention. A significant source of data used to establish basic composition criteria for edible oils and fats of major importance has been the Ministry of Agriculture, Fisheries and Food (MAFF) and the Federation of Oils, Seeds and Fats Associations (FOSFA) at the Leatherhead Food Research Association facility in the United Kingdom. It must also be noted that figures that reference "SDA + Vistive^{Tm}" also comprise the LC-PUFA oil of the food/feed products of the invention.

To establish meaningful standards data, it is essential that sufficient samples be collected from representative geographical origins and that the oils are pure relative to the compositions intended. In the MAFF/FOSFA work, over 600 authentic commercial samples of vegetable oilseeds of known origin and history, generally of ten different geographical origins, were studied for each of 11 vegetable oils. The extracted oils were analyzed to determine their overall fatty acid composition ("FAC"). The FAC at the 2-position of the triglyceride, sterol and tocopherol composition, triglyceride carbon number and iodine value, protein values in the oil, melting point and solid fat content as appropriate are determined.

Prior to 1981, FAC data were not included in published standards because data of sufficient quality was not available. In 1981, standards were adopted that included FAC ranges as mandatory compositional criteria. The MAFF/FOSFA work provided the basis for later revisions to these ranges.

In general, as more data became available, it was possible to propose fatty acid ranges much narrower and consequently more specific than those adopted in 1981. Table la gives examples of FAC of oils that were adopted by the Codex Alimentarius Commission (CAC) in 1981 and ranges for the same oils proposed at the Codex Committee on Fats and Oils (CCFO) meeting held in 1993.

**Table 1a: STANDARDS FOR FATTY ACID COMPOSITION OF OILS**

| Fatty acid | Soybean oil | | Groundnut oil | | Cottonseed oil | | Sunflower-seed oil | |
|---|---|---|---|---|---|---|---|---|
| | 1981 | 1993 | 1981 | 1993 | 1981 | 1993 | 1981 | 1993 |
| C14:0 | < 0.5 | < 0.2 | < 0.6 | < 0.1 | 0.4-2 | 0.6-1 | < 0.5 | < 0.2 |
| C16:0 | 7-14 | 8-13.3 | 6-16 | 8.3-14 | 17-31 | 21.4-26.4 | 3-10 | 5.6-7.6 |
| C16:1 | < 0.5 | < 0.2 | < 1 | < 0.2 | 0.5-2 | 0-1.2 | < 1 | < 0.3 |
| C18:0 | 1.4-5.5 | 2.4-5.4 | 1.3-6.5 | 1.9-4.4 | 1-4 | 2.1-3.3 | 1-10 | 2.7-6.5 |
| C18:1 | 19-30 | 17.7-26.1 | 35-72 | 36.4-67.1 | 13-44 | 14.7-21.7 | 14-65 | 14-39.4 |
| C18:2 | 44-62 | 49.8-57.1 | 13-45 | 14-43 | 33-59 | 46.7-58.2 | 20-75 | 48.3-74 |
| C18:3 | 4-11 | 5.5-9.5 | < 1 | < 0.1 | 0.1-2.1 | 0-0.4 | 0-0.7 | 0-0.2 |
| C20:0 | <1 | 0.1-0.6 | 1-3 | 1.1-1.7 | 0-0.7 | 0.2-0.5 | 0-1.5 | 0.2-0.4 |
| C20:1 | <1 | <0.3 | 0.5-2.1 | 0.7-1.7 | 0-0.5 | 0-0.1 | 0-0.5 | 0-0.2 |
| C22:0 | < 0.5 | 0.3-0.7 | 1-5 | 2.1-4.4 | 0-0.5 | 0-0.6 | 0-1 | 0.5-1.3 |
| C22:1 | - | < 0.3 | <2 | < 0.3 | 0-0.5 | 0-0.3 | 0-0.5 | 0-0.2 |
| C22:2 | | - | - | - | - | - | - | 0-0.3 |
| 024:0 | - | < 0.4 | 0.5-3 | 1.1-2.2 | 0-0.5 | 0-0.1 | 0-0.5 | 0.2-0.3 |
| C24:1 | - | - | - | <0.3 | - | - | <0.5 | - |

| | | | | | | | | |
|---|---|---|---|---|---|---|---|---|
| *Sources:* CODEX ALIMENTARIUS COMMISSION, 1983 and 1993. | | | | | | | | |

Given the above, the LC-PUFA rich oil produced in an recombinant oilseed plant, provides an oil composition not previously available for food manufacturers. It provides for the incorporation of an Omega-3 oil in food products that was not present in appreciable amounts in typical vegetable oils prior to the current invention. In addition the use of this Omega-3 oil is made possible without the traditional concerns with food sensory qualities, or shelf-life when such oils are delivered from a fish or algal source. After delivery of the oil it can be taken and utilized for the production of baked goods, dairy products, spreads, margarines, sports products, nutrition bars and infant formulas, feed, aquaculture, neutraceutical and medicinal uses. Each having enhanced nutritional content.

Turning to Table 1b, to illustrate the utility of the current invention a variety of food products have been/are being chosen representing a broad range of food categories, to determine the impact of LC-PUFA and other Omega-3 oils on product taste and shelf life.

Oxidative stability, as measured by accepted shelf-life sensory tests, is an important PUFA characteristic that determines the useful lifetime and flavor characteristics of fat and oils. Oxidative deterioration in fats and oils can be assessed by wet chemical methods such as peroxide value (PV, which measures peroxides resulting from primary oxidation), and p-anisidine value (AV, which principally measures 2-alkenals resulting from secondary oxidation), or in foods, can be assessed by sensory tasting tests. Selected food categories and products are as follows:

**Table 1b:**

| BEVERAGES | DAIRY PRODUCTS | BAKING | PREPARED FOODS | OIL BASED PRODUCTS | SNACK FOODS |
|---|---|---|---|---|---|
| Soy milks | Cheeses | Breads | Entrees | Salad | Granola |
| Smoothies | Cream | Rolls | Side Dishes | Dressing | Cereals |
| Fruit Juices | Cheeses | Cakes | Soups | Mayonnaise | Snack/Nutritional |
| Dairy Drinks | Sour Cream | Pastries | Sauces | Margarine/ | Bars |
| | Yogurt | Cookies | Processed | Spreads | Confectionary |
| | Yogurt | Crackers | Meats | Shortening | |
| | Drinks | Muffins | Processed | | |
| | Non Dairy | | Fish | | |
| | Creamers | | Pet Foods | | |
| | Dips | | | | |

According to the current studies the development of food products incorporating transgenic LC-PUFA provided several formulations and processes. Additional development and research has been conducted for flavor optimization and the enhancement of shelf-life characteristics. For example, food products of the current invention, include baked goods and baked good mixes (e.g., cakes, brownies, muffins, cookies, pastries, pies, and pie crusts), shortening and oil products (e.g., shortenings, margarines, frying oils, cooking and salad oils, popcorn oils, salad dressings, and mayonnaise), foods that are fried in oil (e.g., potato chips, corn chips, tortilla chips, other fried farinaceous snack foods, french fries, doughnuts, and fried chicken), dairy products and artificial dairy products (e.g., butter, ice cream and other fat-containing frozen desserts, yogurt, and cheeses, including natural cheeses, processed cheeses, cream cheese, cottage cheese, cheese foods and cheese spread, milk, cream, sour cream, buttermilk, and coffee creamer), meat products (e.g., hamburgers, hot dogs, wieners, sausages, bologna and other luncheon meats, canned meats, including pasta/meat products, stews, sandwich spreads, and canned fish), meat analogs, tofu, and various kinds of protein spreads, sweet goods and confections (e.g., candies, chocolates, chocolate confections, frostings, and icings, syrups, cream fillings, and fruit fillings), nut butters and various kinds of soups, dips, sauces and gravies.

Disclosed herein are formulations on target levels of Omega-3 oils for each food product. These levels were identified based on bio-equivalence of the LC-PUFA product. The following information in Table 2a, identifies the targeted Omega 3 levels on a per serving basis:

**Table 2a:**

| Omega-3 Source | mg Omega-3 per serving |
|---|---|
| Stearidonic Acid (SDA) in the LC- PUFA Composition | 375 |
| EPA/DHA (fish/algal oil) | 130 |
| ALA (flax oil) | 320 |

Based on this information, food/feed products were developed with the appropriate level of oil to deliver the targeted levels on a per serving basis. The amount added varied between different applications due to the differences in serving size.

Below are Tables 2b-d reflecting the ranges of the LC-PUFA oil compositions of the food/feed products.

**Table 2b: LC-PUFA Oil Variant-1 (Produced by Transgenic Plants)**

| ANALYTICAL DATA OF SOYBEAN SEEDS AND OILS - CRUSH (250 kg) | | | | | | | | | |
|---|---|---|---|---|---|---|---|---|---|
| | SEED | | | CRUDE OIL | | | RBD OIL | | |
| Moisture (w/w%) | 9.13 | 8.8 | 11.51 | N/A | N/A | N/A | N/A | N/A | N/A |
| Oil Content (%) | 192 | 18.56 | 19.72 | N/A | N/A | N/A | N/A | N/A | N/A |
| Peroxide value (PV, meq/kg) | N/A | N/A | N/A | 0.46 | 0.00 | 0.06 | 0.0 | 0.0 | 0.0 |
| Free fatty acids (FFA, %) | N/A | N/A | N/A | 0.24 | 024 | 0.42 | 0.05 | 0.13 | 0.05 |
| p-Anisidine value (AV) | N/A | N/A | N/A | 0.43 | 0.31 | 0.22 | 0.3 | 0.63 | 0.83 |
| Conjugated dienes (CD) | N/A | N/A | N/A | N/A | N/A | N/A | N/A | N/A | N/A |
| Rancimat@ 110C, hrs | N/A | N/A | N/A | N/A | N/A | N/A | 4.6 | 1.89 | 1.85 |
| *Trans* fatty acids (mg/g) | N/A | N/A | N/A | N/A | N/A | N/A | N/A | N/A | N/A |
| Fatty acid composition (FAC, w/w %) | | | | | | | | | |
| C14:0 (Myristic) | 0.11 | 0.1 | 0.1 | 0.09 | 0.09 | 0.08 | 0.09 | 0.08 | 0.08 |
| C16:0 (Palmitic) | 11.43 | 11.82 | 12.15 | 11.68 | 12.2 | 12 | 11.57 | 11.3 | 12.23 |
| C16:1n7 (Palmitoleic) | 0.1 | 0.09 | 0.09 | 0.1 | 0.12 | 0.14 | 0.1 | 0.09 | 0.14 |
| C18:0(Stearic) | 4.26 | 4.28 | 4.31 | 4.26 | 4.41 | 4.24 | 4.24 | 4.4 | 4.26 |
| C18:1n9(Oleic) | 21.09 | 19.44 | 18.54 | 20.88 | 19.28 | 18.6 | 21.16 | 19.3 | 19.85 |
| C18:1 (Octadecenoic) | 1.47 | 1.52 | 1.50 | 1.46 | 1.48 | 1.46 | 1.46 | 1.52 | 1.44 |
| C18:2n6(Linoleic) | 51.75 | 24.82 | 24.56 | 52.14 | 25.48 | 24.06 | 51.88 | 2538 | 24.1 |
| C18:3n6 (Gamma linolenic) | | 5.28 | 6.17 | | 5.23 | 6.15 | | 5.27 | 621 |
| C18:3n3 (Alpha linolenic) | 8.47 | 10.00 | 10.14 | 8.22 | 10.6 | 10.03 | 823 | 10.72 | 10.15 |
| ***C18:4n3 (Stearidonic)*** | | ***20.40*** | ***20.90*** | | ***19.40*** | ***21.16*** | | ***20.16*** | ***21.10*** |
| C20:0 (Arachidic) | 0.33 | 0.35 | 0.36 | 0.32 | 0.37 | 0.36 | 0.32 | 0.37 | 0.37 |
| C20:1n9 (Eicosenoic) | 0.16 | 0.17 | 0.18 | 0.15 | 0.24 | 0.24 | 0.15 | 0.18 | 0.22 |
| C20:2n6 (Eicosadienoic) | 0.03 | 0.02 | 0.03 | 0.03 | 0.03 | 0.03 | 0.03 | 0.02 | 0.03 |
| C22:0(Behenic) | 031 | 030 | 0.31 | 0.32 | 0.31 | 0.31 | 0.32 | 0.32 | 0.3 |
| C24:0 (Lignoceric) | 0.0.1 | 0.06 | 0.06 | 0.1 | 0.08 | 0.07 | 0.1 | 0.06 | 0.07 |
| Others | 039 | 0.69 | 0.6 | 0.25 | 0.68 | 1.07 | 0 35 | 0.83 | 0.56 |
| Total | 100.0 | 99.3 | 100.0 | 100.0 | 100 | 100.0 | 100.0 | 100.0 | 100.0 |
| | | | | 70Y 3.2R | 70Y3.6R | 70Y3.8R | | | |
| Color (525") | N/A | N/A | N/A | (1") | (1") | (1") | 2.8Y0.1R | 9Y 0.2R | 33Y0.0R |
| Chlorophyl (ppm) | N/A | N/A | N/A | 0.007 | 0.004 | 0.011 | 0.02 | 0.028 | 0.013 |
| Tocopherois (ppm) | | | | | | | | | |
| Alpha | N/A | N/A | N/A | 98.5 | 106 | 101 | 99.4 | 103 | 95.3 |
| Gamma | N/A | N/A | N/A | 940 | 869 | 834 | 914 | 815 | 765 |
| Delta | N/A | N/A | N/A | 305 | 285 | 286 | 293 | 249 | 235 |
| Total | N/A | N/A | N/A | 1343.5 | 1260.0 | 1221.0 | 1306.4 | 1167.0 | 1095.3 |
| Sterols (ppm) | | | | | | | | | |
| Campesterol | N/A | N/A | N/A | 761 | 799 | 677 | 318 | 227 | 588 |
| Stigmasterol | N/A | N/A | N/A | 722 | 684 | 556 | 240 | 130 | 444 |
| Beta-Sitosterol | N/A | N/A | N/A | 1849 | 2196 | 1920 | 1071 | 1021 | 1747 |
| Total | N/A | N/A | N/A | 3332 | 3679 | 3153 | 1629 | 1378 | 2779 |
| Metals (ppm) | | | | | | | | | |
| Phosphorus | N/A | N/A | N/A | 473.6 | 451 | 58.5 | N/A | N/A | N/A |
| Ca | N/A | N/A | N/A | 18.45 | 10.7 | 10.6 | N/A | N/A | N/A |
| Mg | N/A | N/A | N/A | 30.98 | 28.2 | 6.98 | N/A | N/A | N/A |
| Fe | N/A | N/A | N/A | 1.41 | 1.48 | 0.09 | N/A | N/A | N/A |
| Cu | N/A | N/A | N/A | <0.05 | <0.05 | <0.05 | N/A | N/A | N/A |
| Na | N/A | N/A | N/A | 1.75 | 1.39 | <0.20 | N/A | N/A | N/A |

**Table 2c: LC-PUFA Oil Variant-1 (Produced by Transgenic Plants)**

| ANALYTICAL DATA OF SOYBEAN SEEDS AND OILS - CRUSH (5 t Control Soybeans, 6.8 t LC-PUFA soybeans) | | | | | | | | | | | |
|---|---|---|---|---|---|---|---|---|---|---|---|
| | Control (NK43 B1) | SDA | Control (NK43B 1) | SA with N2 | SDA no N2 | Batch 1&2 Combo | Batch 2a | Batch 2b | SDA w N2 Batch 1 | SDA w N2 Batch 2 | SDA w/o N2 |
| | | | | | | | | | | | |
| Moisture, %* or ppm | 12.7* | 12.1* | N/A | N/A | N/A | 45.3 | 22.9 | 16.7 | 99.2 | 107.4 | 115.7 |
| Oil content, % | 19.9 | 20.0 | N/A | N/A | N/A | N/A | N/A | N/A | N/A | N/A | N/A |
| Crude fiber, % | 4.43 | 4.55 | N/A | N/A | N/A | N/A | N/A | N/A | N/A | N/A | N/A |
| Ash, % | 4.68 | 4.63 | N/A | N/A | N/A | N/A | N/A | N/A | N/A | N/A | N/A |
| Urease | 2.16 | 2.14 | N/A | N/A | N/A | N/A | N/A | N/A | N/A | N/A | N/A |
| Protein, (N*6.25)% | 36.0 | 36.0 | N/A | N/A | N/A | N/A | N/A | N/A | N/A | N/A | N/A |
| Trypsin inhibitor | 43,300 | 39,000 | N/A | N/A | N/A | N/A | N/A | N/A | N/A | N/A | N/A |
| Free fatty acids (FFA, %) | N/A | N/A | 0.235 | 0.14 | 0.28 | 0.04 | 0.04 | 0.04 | 0.02 | 0.03 | 0.03 |
| Peroxide value (PV, meq/kg) | N/A | N/A | 0.17 | 0.31 | 0.39 | 0.1 | 0.1 | 0.1 | 0.0 | 0.0 | 0.1 |
| p-Anisidine value (AV) | N/A | N/A | 0.31 | 0.47 | 0.71 | 2.64 | 0.98 | 0.8 | 0.4 | 1.05 | 1.1 |
| Conjugated dienes (CD) | N/A | N/A | N/A | N/A | N/A | N/A | N/A | N/A | N/A | N/A | N/A |
| *Trans* fatty acids, % | 0.00 | 0.00 | 0.19 | 0.46 | 0.48 | 0.31 | 0.29 | 0.30 | 0.89 | 0.92 | 0.86 |
| Fatty acid composition (FAC, w/w %) | | | | | | | | | | | |
| C14:0 (Myristic) | 0.09 | 0.11 | 0.08 | 0.10 | 0.10 | 0.07 | 0.07 | 0.07 | 0.10 | 0.10 | 0.11 |
| C16:0 (Palmitic) | 11.14 | 12.14 | 10.65 | 12.07 | 12.54 | 10.49 | 10.48 | 10.49 | 12.07 | 12.06 | 12.03 |
| C16:1 (*trans-*Hexadecanoic)** | | | 0.01 | 0.01 | 0.01 | 0.01 | 0.01 | 0.01 | 0.01 | 0.01 | 0.01 |
| C16:1n7 (Palmitoleic) | 0.15 | 0.15 | 0.11 | 0.11 | 0.10 | 0.11 | 0.11 | 0.11 | 0.11 | 0.11 | 0.11 |
| C17:0 (Margaric) | 0.10 | 0.10 | 0.00 | 0.00 | 0.00 | N/A | N/A | N/A | N/A | N/A | N/A |
| C18:0 (Stearic) | 4.38 | 4.19 | 4.65 | 4.19 | 4.26 | 4.66 | 4.64 | 4.64 | 4.19 | 4.19 | 4.19 |
| C18:1 (*trans*-Octadecenoic) | | | 0.08 | 0.08 | 0.08 | 0.09 | 0.09 | 0.09 | 0.07 | 0.06 | 0.08 |
| C18:1n9 (Oleic) | 20.40 | 18.35 | 20.64 | 17.92 | 17.91 | 20.70 | 20.66 | 20.68 | 17.92 | 17.92 | 17.96 |
| C18:1 (Octadecenoic) | 1.29 | 1.27 | 1.47 | 1.47 | 1.49 | 1.49 | 1.50 | 1.48 | 1.46 | 1.47 | 1.46 |
| C18:2 (*trans-*Octadecadienoic) | | | 0.05 | 0.09 | 0.09 | 0.09 | 0.10 | 0.10 | 0.13 | 0.12 | 0.14 |
| C18:2n6 (Linoleic) | 53.51 | 35.07 | 53.10 | 35.22 | 35.34 | 53.07 | 53.07 | 53.07 | 35.21 | 35.26 | 35.47 |
| C18:3 (trans-Octadecatrienoic) | | | 0.04 | 0.18 | 0.20 | 0.13 | 0.10 | 0.11 | 0.40 | 0.42 | 0.36 |
| C18:3n6 (Gamma linolenic) | 0.00 | 4.92 | 0.00 | 4.95 | 4.82 | N/A | N/A | N/A | 4.91 | 4.90 | 4.83 |
| C18:3n3 (Alpha linolenic) | 7.34 | 10.31 | 7.63 | 10.27 | 10.18 | 7.58 | 7.63 | 7.62 | 10.13 | 10.11 | 10.09 |
| C18:4 (*trans-*Octadecatetraenoic) | | | 0.00 | 0.11 | 0.10 | N/A | N/A | N/A | 0.28 | 0.31 | 0.27 |
| *C18:4n3 (Stearidonic)* | *0*.*00* | *11.70* | *0*.*00* | *11.78* | *11.31* | N/A | N/A | N/A | *11.43* | *11.37* | *11.25* |
| C20:0 (Arachidic) | 0.38 | 0.39 | 0.39 | 0.42 | 0.41 | 0.38 | 0.39 | 0.39 | 0.41 | 0.41 | 0.41 |
| C20:1n9 (Eicosenoic) | 0.27 | 0.28 | 0.21 | 0.25 | 0.23 | 0.21 | 0.21 | 0.21 | 0.36 | 0.36 | 0.36 |
| C20:2n6 (Eicosadienoic) | 0.04 | 0.04 | 0.00 | 0.00 | 0.00 | 0.00 | 0.00 | 0.00 | 0.00 | 0.00 | 0.00 |
| C22:0 (Behenic) | 0.38 | 0.33 | 0.40 | 0.33 | 0.34 | 0.41 | 0.40 | 0.40 | 0.35 | 0.35 | 0.36 |
| C24:0 (Lignoceric) | 0.16 | 0.14 | 0.14 | 0.13 | 0.13 | 0.14 | 0.14 | 0.14 | 0.13 | 0.13 | 0.13 |
| Others | 0.39 | 0.53 | 0.35 | 0.32 | 0.34 | 0.38 | 0.39 | 0.38 | 0.35 | 0.35 | 0.37 |
| Total | 100.0 | 100.0 | 100.0 | 100.0 | 100.0 | 100.0 | 100.0 | 100.0 | 100.0 | 100.0 | 100.0 |
| | | | | | | 2.6Y | 1.2Y | 0.9Y | 1.4Y | | 3Y |
| Color (5.25")** | N/A | N/A | N/A | N/A | N/A | 0.2R | 0.0R | 0.0R | 0.0R | 6.5Y 0.3R | 3Y 0.4R |
| Chlorophyll, ppm | N/A | N/A | N/A | N/A | N/A | 0.0 | 0.0 | 0.0 | 0.0 | 0.0 | 0.0 |
| Citric acid, ppm | N/A | N/A | N/A | N/A | N/A | <10 | <10 | <10 | <10 | <10 | <10 |
| Tocopherols (ppm) | | | | | | | | | | | |
| Alpha | N/A | N/A | N/A | N/A | N/A | 90.7 | 84.6 | 87.4 | 151 | 157 | 139 |
| Gamma | N/A | N/A | N/A | N/A | N/A | 727 | 725 | 689 | 683 | 721 | 650 |
| Delta | N/A | N/A | N/A | N/A | N/A | 159 | 171 | 162 | 102 | 104 | 105 |
| Total | N/A | N/A | N/A | N/A | N/A | 976.7 | 980.6 | 938.4 | 936 | 982 | 894 |
| Sterols (ppm) | | | | | | | | | | | |
| campesterol | N/A | N/A | N/A | N/A | N/A | 533 | 459 | 451 | 460 | 495 | 383 |
| stigmasterol | N/A | N/A | N/A | N/A | N/A | 569 | 453 | 448 | 465 | 519 | 364 |
| B-sitosterol | N/A | N/A | N/A | N/A | N/A | 1550 | 1410 | 1380 | 1620 | 1680 | 1480 |
| Other | N/A | N/A | N/A | N/A | N/A | 465 | 398 | 403 | 536 | 581 | 472 |
| Total | N/A | N/A | N/A | N/A | N/A | 3117 | 2720 | 2682 | 3081 | 3275 | 2699 |
| Metals (ppm) | | | | | | | | | | | |
| Phosphorus | N/A | N/A | N/A | N/A | N/A | N/A | N/A | N/A | N/A | N/A | N/A |
| Ca | N/A | N/A | N/A | N/A | N/A | N/A | N/A | N/A | N/A | N/A | N/A |
| Cu | N/A | N/A | N/A | N/A | N/A | N/A | N/A | N/A | N/A | N/A | N/A |
| Fe | N/A | N/A | N/A | N/A | N/A | N/A | N/A | N/A | N/A | N/A | N/A |
| Mg | N/A | N/A | N/A | N/A | N/A | N/A | N/A | N/A | N/A | N/A | N/A |
| Na | N/A | N/A | N/A | N/A | N/A | N/A | N/A | N/A | N/A | N/A | N/A |

**TABLE 2d. LC-PUFA Oil Variant-1 (Produced by Transgenic Plants)**

| ANALYTICAL DATA OF SOYBEAN SEEDS AND OILS - CRUSH (5 t Control Soybeans, 6.8 t LC-PUFA soybeans) | | | | | | | | | |
|---|---|---|---|---|---|---|---|---|---|
| | Control Seed | | | SDA Seed | Crude Oil | | RBD Oil | | |
| | RR1 | A3525 | MO591 | SDA Comp | Control | Avg. SDA Values | AVG Control Values | Lt Bleach SDA | Hvy Bleach-SDA |
| Moisture (w/w % or ppm*) | 11.54 | 10.2 | 10.24 | | | | 33.4* | 38.6* | 55.45* |
| Oil content (%) | 18.90 | 19.59 | 19.28 | 19.08 | | | | | |
| Peroxide value (PV, meq/kg) | 0.3 | 0.46 | 0.5 | 0.5 | 0.21 | 0.26 | 0.0 | 0.0 | 0.0 |
| Free fatty acids (FFA, %) | 0.44 | 0.11 | 0.15 | 0.27 | 0.3 | 0.4 | 0.03 | 0.04 | 0.03 |
| p-Anisidine value (AV) | N/A | N/A | N/A | N/A | 0.34 | 1.63 | 1.07 | 2.35 | 2.05 |
| Conjugated dienes (CD) | N/A | N/A | N/A | N/A | N/A | N/A | N/A | N/A | N/A |
| Trans fatty acids (w/w %) | N/A | N/A | N/A | N/A | 0.19 | 0.48 | 0.32 | 0.63 | 0.67 |
| Fatty acid composition (FAC, w/w %) | | | | | | | | | |
| C14:0 (Myristic) | 0.9 | 0.10 | 0.10 | 0.10 | 0.08 | 0.09 | 0.07 | 0.08 | 0.08 |
| C16:0 (Palmitic) | 10.94 | 11.41 | 11.71 | 12.68 | 11.11 | 12.59 | 10.99 | 12.42 | 12.42 |
| C16:1 (*Trans*-Hexadecanoic) | N/A | N/A | N/A | 0 | 0.01 | 0.01 | 0.01 | 0.01 | 0.01 |
| C16:1n7 (Palmitoleic) | 0.15 | 0.15 | 0.15 | 0.16 | 0.11 | 0.13 | 0.12 | 0.11 | 0.13 |
| C17:0 (Margaric) | 0.10 | 0.11 | 0.11 | 0.11 | N/A | N/A | 0 | 0 | 0 |
| C18:0 (Stearic) | 4.55 | 4.48 | 4.47 | 4.35 | 4.51 | 4.29 | 4.48 | 4.28 | 4.28 |
| C18:1 (*Trans*-Octadecenoic) | N/A | N/A | N/A | 0 | 0.08 | 0.08 | 0.08 | 0.07 | 0.06 |
| C18:1n9 (Oleic) | 21.70 | 20.90 | 20.51 | 18.47 | 20.77 | 17.76 | 20.82 | 17.83 | 17.85 |
| C18:1 (Octadecenoic) | 0.96 | 1.14 | 1.09 | 1.11 | 1.51 | 1.58 | 1.49 | 1.56 | 1.57 |
| C18:2 (*Trans*-Octadecadienoic) | N/A | N/A | N/A | 0 | 0.06 | 0.08 | 0.10 | 0.08 | 0.10 |
| C18:2n6 (Linoleic) | 51.76 | 52.25 | 52.52 | 31.25 | 52.00 | 31.39 | 52.08 | 31.31 | 31.32 |
| C18:3 (*Trans*-Octadecatrienoic) | N/A | N/A | N/A | 0 | 0.07 | 0.25 | 0.16 | 0.29 | 0.30 |
| C18:3n6 (Gamma linolenic) | 0 | 0.06 | 0 | 5.04 | N/A | 5.10 | 0 | 5.12 | 5.13 |
| C18:3n3 (Alpha linolenic) | 8.29 | 7.91 | 8.03 | 10.50 | 8.15 | 10.48 | 8.09 | 10.41 | 10.38 |
| C18:4 (*trans-*Octadecatetraenoic) | N/A | N/A | N/A | 0 | N/A | 0.13 | 0 | 0.21 | 0.24 |
| *C18:4n3 (Stearidonic)* | N/A | 0.16 | N/A | 14.59 | N/A | 14.64 | 0 | 14.77 | 14.68 |
| C20:0 (Arachidic) | 0.39 | 0.36 | 0.37 | 0.40 | 0.38 | 0.38 | 0.37 | 0.38 | 0.38 |
| C20:1n9 (Eicosenoic) | 0.26 | 0.25 | 0.24 | 0.29 | 0.24 | 0.26 | 0.22 | 0.27 | 0.28 |
| C20:2n6 (Eicosadienoic) | 0.04 | 0.04 | 0.04 | 0.03 | 0.04 | 0.03 | 0.04 | 0.04 | 0.05 |
| C22:0 (Behenic) | 0.41 | 0.34 | 0.34 | 0.33 | 0.38 | 0.32 | 0.37 | 0.34 | 0.34 |
| C24:0 (Lignoceric) | 0.14 | 0.13 | 0.12 | 0.11 | 0.13 | 0.09 | 0.13 | 0.10 | 0.10 |
| Others | 0.21 | 0.22 | 0.20 | 0.49 | 0.39 | 0.33 | 0.39 | 0.31 | 0.31 |
| Total | 100.0 | 100.0 | 100.0 | 100.0 | 100.0 | 100.0 | 100.0 | 100.0 | 100.0 |
| | | | | | 70Y 2.9R | 70Y 3.7R | 5.2Y | 5.5Y | |
| Color (5.25") | N/A | N/A | N/A | N/A | N/A | 0.2R | 0.0R | 0.0R | 0.0R |
| Chlorophyll (ppm) | N/A | N/A | N/A | N/A | 0.156 | 0.0 | 0.0 | 0.0 | 0.0 |
| Citric acid (ppm) | N/A | N/A | N/A | N/A | N/A | <10 | <10 | <10 | <10 |
| Tocopherols (ppm) | | | | | | | | | |
| Alpha | N/A | N/A | N/A | N/A | 96.1 | 111 | 87.6 | 106 | 94.9 |
| Gamma | N/A | N/A | N/A | N/A | 830 | 860 | 723 | 777 | 738 |
| Delta | N/A | N/A | N/A | N/A | 238 | 221 | 183 | 176 | 163 |
| Total | N/A | N/A | N/A | N/A | 1164.1 | 1192 | 993.6 | 1059 | 995.9 |
| Sterols (ppm) | | | | | | | | | |
| Campesterol | N/A | N/A | N/A | N/A | 778 | 668 | 674 | 532 | 498 |
| Stigmasterol | N/A | N/A | N/A | N/A | 773 | 673 | 656 | 512 | 476 |
| Beta-Sitosterol | N/A | N/A | N/A | N/A | 1860 | 1880 | 1700 | 1640 | 1570 |
| Others | N/A | N/A | N/A | N/A | 577 | 732 | 498 | 623 | 599 |
| Total | N/A | N/A | N/A | N/A | 3988 | 3953 | 3528 | 3307 | 3143 |
| Metals (ppm) | | | | | | | | | |
| Phosphorus | N/A | N/A | N/A | N/A | 330 | 756 | N/A | N/A | N/A |
| Ca | N/A | N/A | N/A | N/A | 18.6 | 52.8 | N/A | N/A | N/A |
| Mg | N/A | N/A | N/A | N/A | 23.6 | 47 | N/A | N/A | N/A |
| Fe | N/A | N/A | N/A | N/A | 0.67 | 0.59 | N/A | N/A | N/A |
| Cu | N/A | N/A | N/A | N/A | <0.05 | < | N/A | N/A | N/A |
| Na | N/A | N/A | N/A | N/A | <0.02 | < | N/A | N/A | N/A |

**Table 2e: Finished Base Oil Comparison**

| | SDA | Vistive Oil | Standard Soybean Oil |
|---|---|---|---|
| | | w/o Transgenic oils | |
| Fatty Acid Composition. | | | |
| C14:0 Myristic Acid | 0.1 | 0.08 | 0.06 |
| C16:0 Palmitic Acid | 12.05 | 9.01 | 10.07 |
| C16:1 Palmitoleic Acid | 0.11 | 0.11 | 0.10 |
| C18:0 Stearic Acid | 4.19 | 4.20 | 4.35 |
| C18:1 Oleic Acid | 17.93 | 29.25 | 23.60 |
| C18:2 Linoleic Acid | 35.31 | 52.90 | 52.47 |
| C18:3 Linolenic Acid | 10.11 | 2.55 | 6.69 |
| 18:3 Gamma LA | 4.88 | | |
| C18:4 Stearidonic Acid | 11.35 | | |
| C20:0 Arachidic Acid | 0.41 | 0.31 | 0.34 |
| C20:1 | 0.36 | 0.31 | 0.27 |
| C22:0 Behenic Acid | 0.35 | 0.35 | 0.35 |
| C24:0 | 0.13 | 0.10 | 0.10 |
| | | | |
| % Total Trans Fatty Acid | 0.89 | 1.15 | 16.53 |

| | | | |
|---|---|---|---|
| *The LC-PUFA oil of the food/feed product is a mixture of the transgenic oil "SDA" and Vistive Oil. | | | |

The primary source of stearidonic acid was oil extracted from transgenic soybeans which have been engineered to produce high levels of stearidonic acid. The soybeans were processed at an oil processing facility and oil was extracted consistent with the methods described in US Patent Applications Publications 2006/0111578, and 2006/0111254.

To make the LC-PUFA composition, an amount of transgenically derived SDA oil was used and any liquid soybean oil was replaced with Vistive™ oil. This oil retained the benefits of an SDA rich Omega-3 oil with many of the consistency improvements otherwise found in Vistive™ oils.

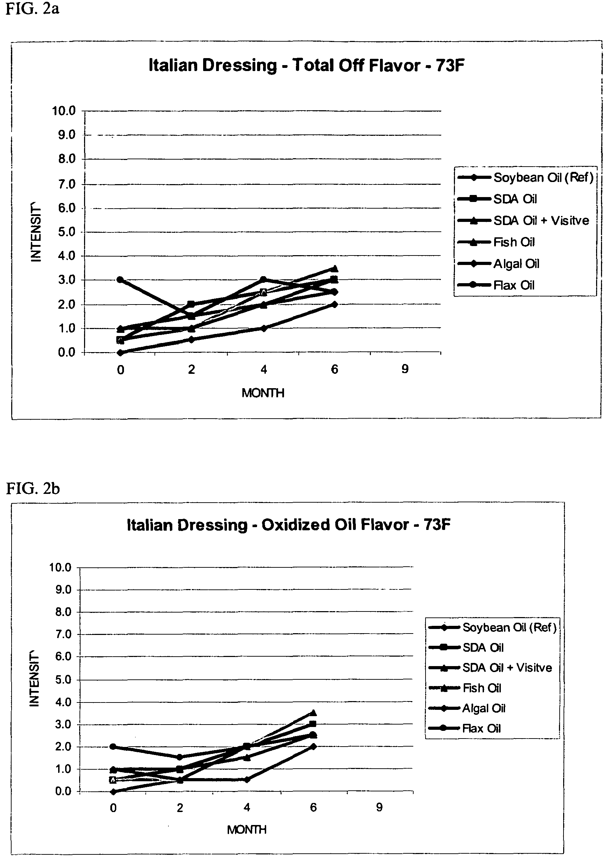

In addition to oil, flour was made from the transgenic and control soybeans typical of industry practices in processing full-fatted soy flour. One example of a food formulation utilizing the LC-PUFA is found in Table 3a-3c, and Figures 2a-2e below. Generally attributes of Italian style dressings are provided in Tables 4a-4c.

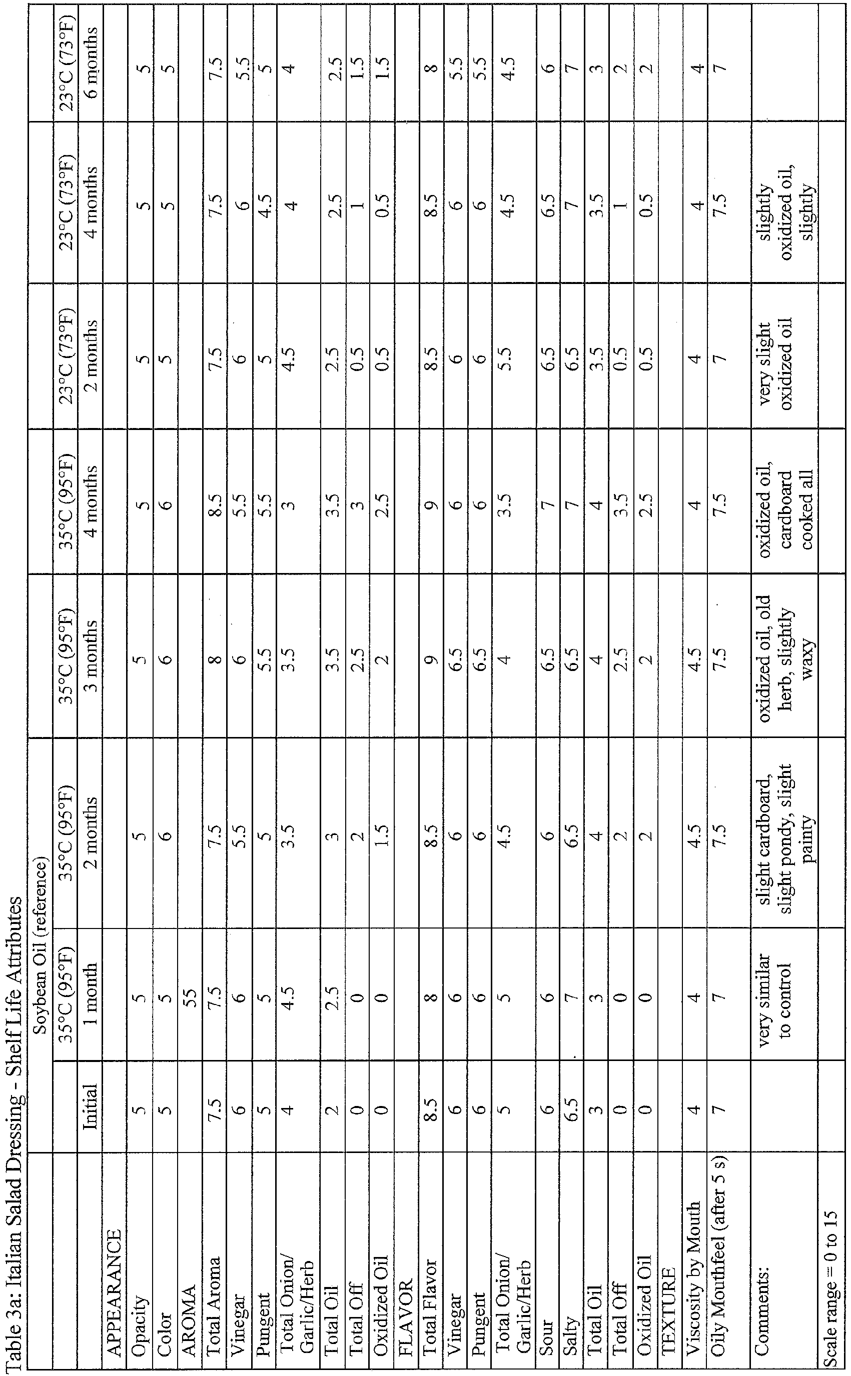

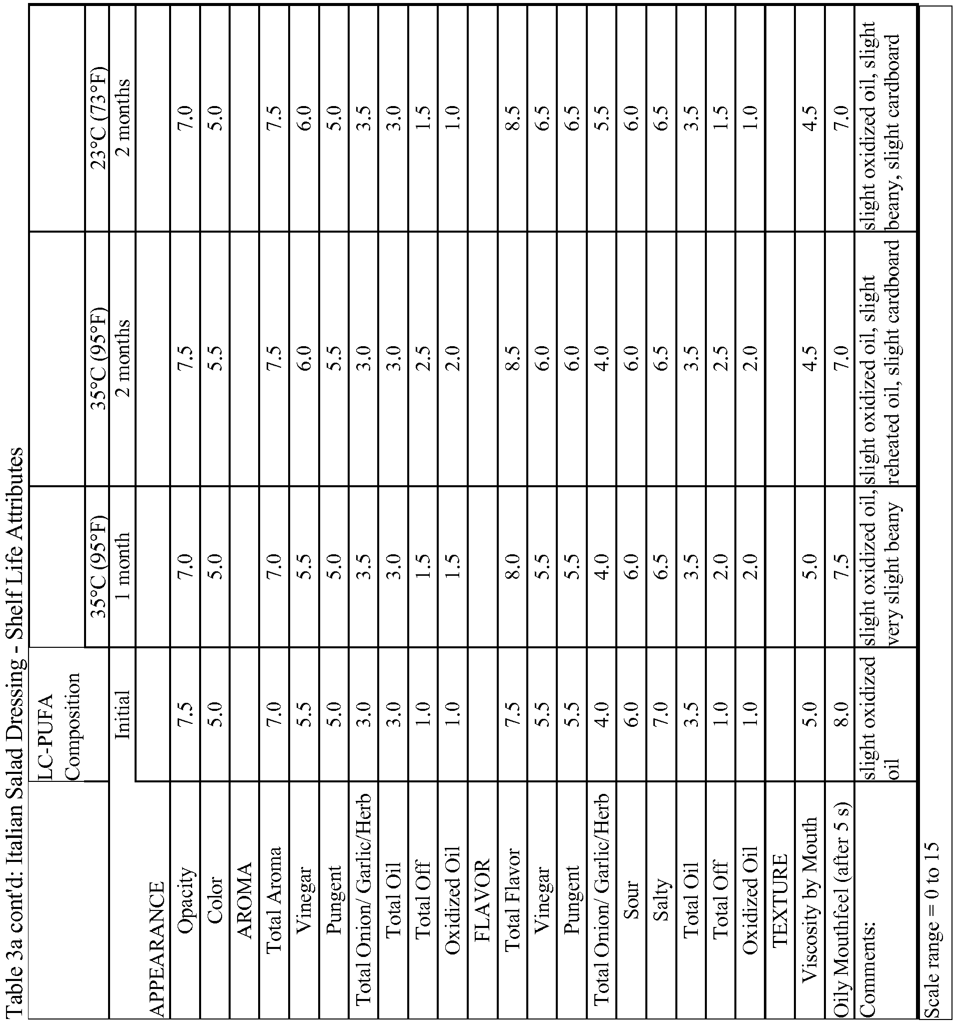

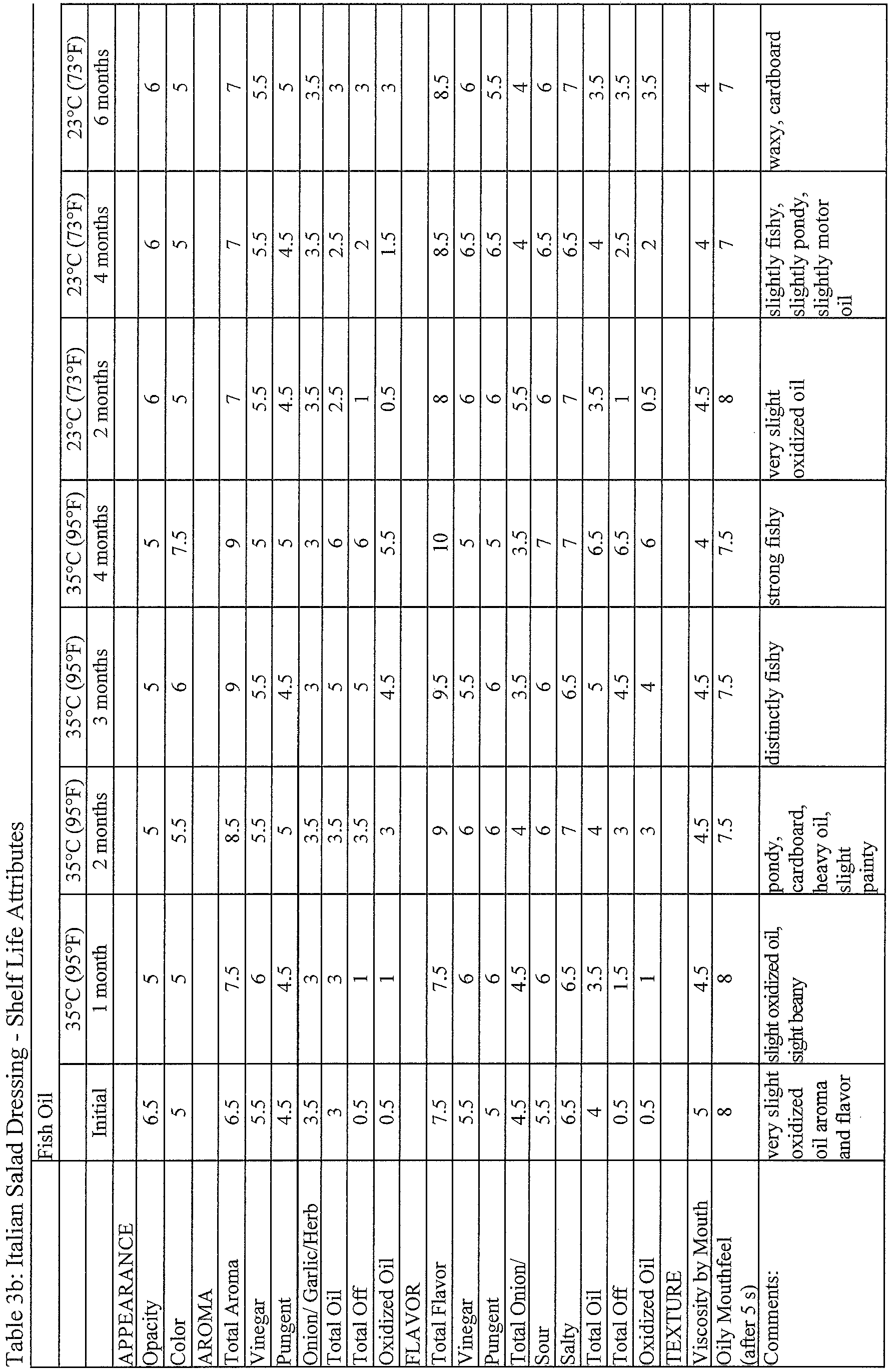

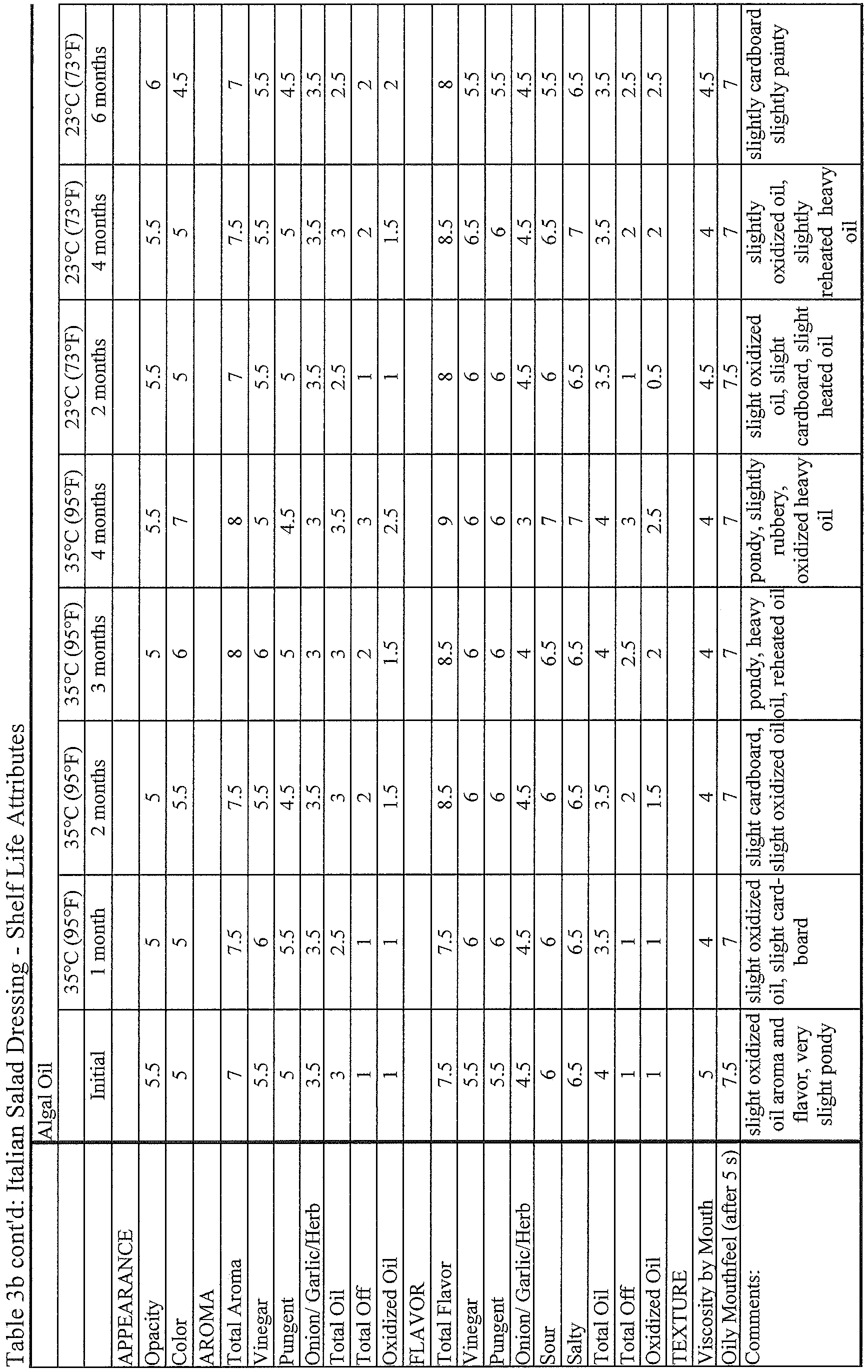

**Table 3c: Italian Salad Dressing - Shelf Life Attributes**

| | Flax Oil | | | | | | | |
|---|---|---|---|---|---|---|---|---|
| | | 35°C | 35°C (95°F) | 35°C (95°F) | 35°C (95°F) | 23°C (73°F) | 23°C (73°F) | 23°C (73°F) |
| | Initial | 1 months | 2 months | 3 months | 4 months | 2 months | 4 months | 6 months |
| APPEARANCE | | | | | | | | |
| Opacity | 5.5 | 5 | 5 | 6 | 5.5 | 5.5 | 5 | 5.5 |
| Color | 5 | 5 | 5.5 | 6 | 7 | 5 | 5 | 5 |

| AROMA | | | | | | | | |
|---|---|---|---|---|---|---|---|---|
| Total Aroma | 7 | 7 | 7.5 | 8 | 8 | 7 | 7 | 7 |
| Vinegar | 5.5 | 6 | 6 | 6 | 6 | 6 | 5.5 | 5.5 |
| Pungent | 5 | 5 | 5 | 5.5 | 5.5 | 4.5 | 4 | 5 |
| Total Onion/ Garlic/Herb | 3.5 | 4 | 3.5 | 3 | 3 | 3.5 | 4 | 3.5 |
| Total Oil | 3.5 | 3 | 3 | 3 | 3.5 | 3 | 3 | 3 |
| Total Off | 2 | 1.5 | 2.5 | 2.5 | 3 | 1.5 | 2.5 | 2.5 |
| Oxidized Oil | 1.5 | 1 | 2.5 | 2 | 2.5 | 1 | 1.5 | 2 |

| FLAVOR | | | | | | | | |
|---|---|---|---|---|---|---|---|---|
| Total Flavor | 8 | 8 | 8.5 | 9 | 9 | 8 | 9 | 8.5 |
| Vinegar | 6 | 5.5 | 6 | 6.5 | 6 | 6 | 6 | 5.5 |
| Pungent | 5.5 | 5.5 | 6 | 6 | 6 | 6 | 6 | 5.5 |
| Total Onion/ Garlic/Herb | 4 | 5 | 4.5 | 4 | 3.5 | 5 | 5 | 4.5 |
| Sour | 6 | 5.5 | 6 | 6.5 | 6.5 | 5.5 | 6.5 | 5.5 |
| Salty | 6.5 | 6.5 | 6.5 | 6.5 | 7 | 6.5 | 7 | 6.5 |
| Total Oil | 4 | 4 | 4 | 3.5 | 4 | 4 | 4 | 3.5 |
| Total Off | 3 | 1.5 | 2.5 | 2 | 3.5 | 1.5 | 3 | 2.5 |
| Oxidized Oil | 2 | 0.5 | 2 | 2 | 2.5 | 1.5 | 2 | 2.5 |

| TEXTURE | | | | | | | | |
|---|---|---|---|---|---|---|---|---|
| Viscosity by Mouth | 5 | 4.5 | 4.5 | 4 | 4 | 5 | 4.5 | 4 |
| Oily Mouthfeel (after 5 s) | 8 | 7.5 | 7.5 | 7.5 | 7 | 7.5 | 7.5 | 7 |

**Table 4a: ITALIAN SALAD DRESSING**

| ***LC-PUFA SALAD DRESSING FORMULATIONS*** - ***ITALIAN*** | | | | | | |
|---|---|---|---|---|---|---|
| | | | | | | |
| Variant | Control | LC-PUFA | SDA | Fish Oil | Algal Oil | Flax Oil |
| Formula Number | 50-RA-325-000 | 50-RA-691-000 | 50-RA-326-000 | 50-RA-328-000 | 50-RA-330-000 | 50-RA-327-000 |
| | | | | | | |
| INGREDIENT | % | | | | | |
| Liquid Soybean Oil | 44.5000 | 33.17 | 33.1700 | 43.0700 | 43.2700 | 42.9700 |
| | | | | | | |
| Omega 3 Oil | | 11.33 | 11.33 | 1.43 | 1.23 | 1.53 |
| Water | 39.3530 | 39.3530 | 39.3530 | 39.3530 | 39.3530 | 39.3530 |
| Egg Yolk, Liquid, 10% Salt | 2.9000 | 2.9000 | 2.9000 | 2.9000 | 2.9000 | 2.9000 |
| Vinegar, White Distilled, 120 g | 2.8500 | 2.8500 | 2.8500 | 2.8500 | 2.8500 | 2.8500 |
| Sugar, White, Fine Granulated | 2.5000 | 2.5000 | 2.5000 | 2.5000 | 2.5000 | 2.5000 |
| Buttermilk Powder, Cultured LOL#20631 | 2.1000 | 2.1000 | 2.1000 | 2.1000 | 2.1000 | 2.1000 |
| Salt, Regular, Non Iodized | 1.7000 | 1.7000 | 1.7000 | 1.7000 | 1.7000 | 1.7000 |
| Flavor, Cultured Buttermilk, Cargill#24521 | 1.5000 | 1.5000 | 1.5000 | 1.5000 | 1.5000 | 1.5000 |
| Garlic, Dehydrated, Granular | 0.4500 | 0.4500 | 0.4500 | 0.4500 | 0.4500 | 0.4500 |
| Onion, Dehydrated, Granular | 0.4400 | 0.4400 | 0.4400 | 0.4400 | 0.4400 | 0.4400 |
| Mustard Flour, Wisconsin Spice SP448 | 0.4000 | 0.4000 | 0.4000 | 0.4000 | 0.4000 | 0.4000 |
| Acid, Phosphoric, 75% | 0.4000 | 0.4000 | 0.4000 | 0.4000 | 0.4000 | 0.4000 |
| Gum, Xanthan, 60 mesh, Regular | 0.2750 | 0.2750 | 0.2750 | 0.2750 | 0.2750 | 0.2750 |
| Preservative, Potassium Sorbate | 0.2000 | 0.2000 | 0.2000 | 0.2000 | 0.2000 | 0.2000 |
| Monosodium Glutamate (MSG) | 0.2000 | 0.2000 | 0.2000 | 0.2000 | 0.2000 | 0.2000 |
| Preservative, Sodium Benzoate, Granular | 0.1000 | 0.1000 | 0.1000 | 0.1000 | 0.1000 | 0.1000 |
| Pepper, Black, 30-60 mesh | 0.1000 | 0.1000 | 0.1000 | 0.1000 | 0.1000 | 0.1000 |
| Parsley, Dehydrated, Granular -10 +30 | 0.0250 | 0.0250 | 0.0250 | 0.0250 | 0.0250 | 0.0250 |
| Preservative, EDTA, Calcium Disodium | 0.0070 | 0.0070 | 0.0070 | 0.0070 | 0.0070 | 0.0070 |
| TOTAL | 100.0000 | 100.0000 | 100.0000 | 100.0000 | 100.0000 | 100.0000 |

**Table 4b: ITALIAN SALAD DRESSING**

| ***Italian Salad Dressing Production Process:*** | |
|---|---|
| 1. | Check that the mixer is in good working condition, free and clear of dust & dirt, seated tight, mill set correctly. |
| 2. | Set mix tank speed to 25 Hz. |
| 3. | Meter in water to mix tank. |
| 4. | Add preservatives (Benzoate, Sorbate, EDTA) into mix tank. |
| 5. | Make gum slurry (Xanthan Gum + 400 g soybean oil). |
| 6. | Add to Dixie tank, mix for 3 min |
| 7 | Add the rest of the dry ingredients to the Dixie mill. |
| 8. | Adjust mix tank speed to 45 Hz. |
| 9. | Add HFCS, caramel color, and Yellow No. 6 to the Dixie tank. |
| 10. | Slowly add remainder of soybean oil and if appropriate, Omega 3 oil. |
| 11. | Add distilled vinegar, mix for 30 s. |
| 12. | Open mix tank valve, and set pump speed to 30 Hz. |
| 13. | Turn on pum to pack; colloid mill is off. |
| 14. | Pack into bulk or individual containers, cap. |

**Table 4c: ITALIAN SALAD DRESSING SHELF LIFE PRODUCTION ANALTYICAL/MICRO RESULTS ITALIAN DRESSING**

| | Control | LC-PUFA | SDA | Fish Oil | Algal Oil | Flax Oil |
|---|---|---|---|---|---|---|
| | 50-RA-252-000 | 50-RA-690-000 | 50-RA-248-000 | 50-RA-264-000 | 50-RA-266-000 | 50-RA-265-000 |
| PH | 3.51 | 3.52 | 3.52 | 3.53 | 3.52 | 3.51 |
| Total Acidity | 1.01 | 1.02 | 1.02 | 1.00 | 1.01 | 1.02 |
| Total Solids | 2.56 | 2.56 | 2.51 | 2.50 | 2.52 | 2.53 |
| Bostwick (viscosity) | 18.9 cm | 19.25 cm | 19.1 cm | 1925 cm | 19.0 cm | 18.9 cm |
| Total Plate Count | <10 | <10 | <10 | <10 | <10 | <10 |
| Lactics | <10 | <10 | <10 | <10 | <10 | <10 |
| Yeast | <10 | <10 | <10 | <10 | <10 | <10 |
| Mold | <10 | <10 | <10 | <10 | <10 | <10 |

According to the methods disclosed herein samples of various salad dressings were submitted to a contracting food laboratory for confirmatory studies and analysis. The general approach to the shelf-life testing is for 5 attribute panelists to taste the dressings and come to consensus regarding the attributes and intensity (on a 15 pt scale - 0 being absent, 15 being extreme) for each dressing. The lists of attributes identified by the panelists are in the attached documents. Additional attributes are identified as warranted. The characteristics of attribute testing are provided below, Table 5, along with the data from sensory testing at various time points, Table 6.

The underlying VISTIVE soybean oil, developed through conventional breeding, contains less than three percent by weight linolenic acid based on the total weight of fatty acids as compared to the typical eight percent level found in traditional soybeans. The result is a more stable soybean oil, with less need for hydrogenation. Because soybeans with a lower linolenic acid level reduce the need for partial hydrogenation, their application in processed soybean oils will reduce the presence of trans fats in processed soybean oil. In a synergistic combination with the transgenic SDA of the invention a LC-PUFA oil composition has been developed that satisfies both government regulatory needs and commercial needs for dietary oils with a healthier profile. It maintains the lower level of linolenic acid while providing the benefits of Omega-3 oil and enhanced tocopherol levels.

**Table 5: LC-PUFA DRESSING DEFINITIONS OF SENSORY ATTRIBUTES**

| APPEARANCE | |
|---|---|
| Yellow Color | The intensity of the yellow color in the sample, from light to dark yellow. |

| AROMA/FLAVOR | |
|---|---|
| Total Aroma | The total aroma intensity of the sample. |
| Total Flavor | The total flavor intensity of the sample, including the basic tastes. |
| Total Oil | The intensity of aroma/flavor of any type of oil, including oxidized oil. |
| Oxidized Oil | The intensity of aroma/flavor of oxidized oil, described as old oil that has undergone oxidation, characterized as cardboard, beany, painty, or fishy. |
| Total Off Aroma/Flavor | The intensity of aroma/flavor of believed to not intended in the product, includes oxidized oil and other off notes. The nature of the off note is to be described. |
| Mayonnaise/Dairy | The intensity of the aroma/flavor associated with mayonnaise or dairy product. |
| Vinegar | The intensity of the aroma/flavor of white vinegar or acetic acid. |
| Onion/Garlic/Herb | The intensity of aroma/flavor associated with onion, garlic, and all dried and fresh green herbs. |
| Sour | One of the four basic tastes, perceived primarily on the sides of the tongue; common to acids. |
| Salty | One of the four basic tastes, perceived primarily on the sides of the tongue; common to sodium chloride (table salt). |

| FEELING FACTORS | |
|---|---|
| Pungent | The amount of burning or irritation of the nasal cavity produced by smelling the sample, such as with horseradish. |

| TEXTURE | |
|---|---|
| Viscosity by Mouth | The degree of thickness of the sample as perceived when manipulated in the mouth. |
| Oily Mouthcoating | The amount of coating perceived on the soft tissues of the mouth |

| AFTERTASTE | |
|---|---|
| Total Aftertaste | The total aftertaste intensity of the sample. |

### EXAMPLE 1: Salad Dressing

The tables above represent the data developed for a preferred embodiment of the current invention. Please also see Figures 2a-2e for graphical representation of the data out to four months. According to the data provided herein, the samples containing LC-PUFA are signifycantly less off-flavored than corresponding fish and algal Omega-3 oil formulations, providing the benefit of the presence of an omega-3 formulation without the substantially shortened shelf-life and limited stability. Due to pungent flavors and extremely unpleasant odors the fish and algal derived oils simply could not be tested and were removed from the 3 months accelerated evaluation period whereas the LC-PUFA composition was not. Overall the LC-PUFA compositions of the food/feed products demonstrate improved stability, reduced degradation and consequent enhanced shelf-life for commercial utilization in conjunction with the delivery of beneficial Omega-3's into the diet.

With regard to specific salad dressings, the LC-PUFA compositions of the food/feed products developed utilized for enhanced Ranch Dressings maintained their flavor profile longer than the fish and algal oils after 6 months room temperature storage. For Italian dressings, the more complex flavor system does do some masking, but the LC-PUFA containing dressings are again less off flavored than comparable based fish/algal dressings.

### Italian Salad Dressings:

The shelf-life studies, at room temperature and accelerated studies, were completed through 4 months. Each sample has been evaluated by the trained attribute panel in a food laboratory at 0, 2 and 4 months at room temperature and at 1 and 2 months accelerated temperature (35°C (95°F)). For Ranch Dressings, the fish and algal oil samples were only smelled at 3 months due to high off flavor and character at the two month point and were untestable after that point. All other samples, including those containing the LC-PUFA oil, were evaluated at 2 months. This is typical for accelerated shelf life evaluations.

According to the methods disclosed herein the Italian dressings have demonstrated significant stability in terms of flavor relative to other omega-3 containing test subjects. Accelerated testing has been completed through four months testing at 35°C (95°F). At this point, all of the products exhibited off flavors, with the fish oils demonstrating the highest in off notes. Significantly, the LC-PUFA formulations of the food/feed products were very similar to the soybean oil reference with the improvements in composition and health profile in place.

According to the methods disclosed herein the Ranch-style dressings demonstrated significant improvements according to sensory parameters relative to Fish Oil and Algal Oil formulations containing other Omega-3's. Also, accelerated testing has been completed. High intensity off flavors developed in the fish and algal samples at two months whereas the LC-PUFA oil of the food/feed products of the invention and the reference soybean oil could be evaluated according to sensory parameters at 3 months. The reference and flax samples exhibited more characteristic flavors and less off flavor than the LC-PUFA oil of the food/feed products of the invention. The LC-PUFA oil of the food/feed products exhibited more characteristic flavors and less off flavors than the fish and algal samples. This demonstrates that LC-PUFA has improved shelf life vs. fish and algal oils. In addition, room temperature testing was completed for the formulations through 4 months. Results indicate that the LC-PUFA product has a significantly lower profile for off flavors and unpleasant odors relative to other omega-3 sources, including fish and algal oils.

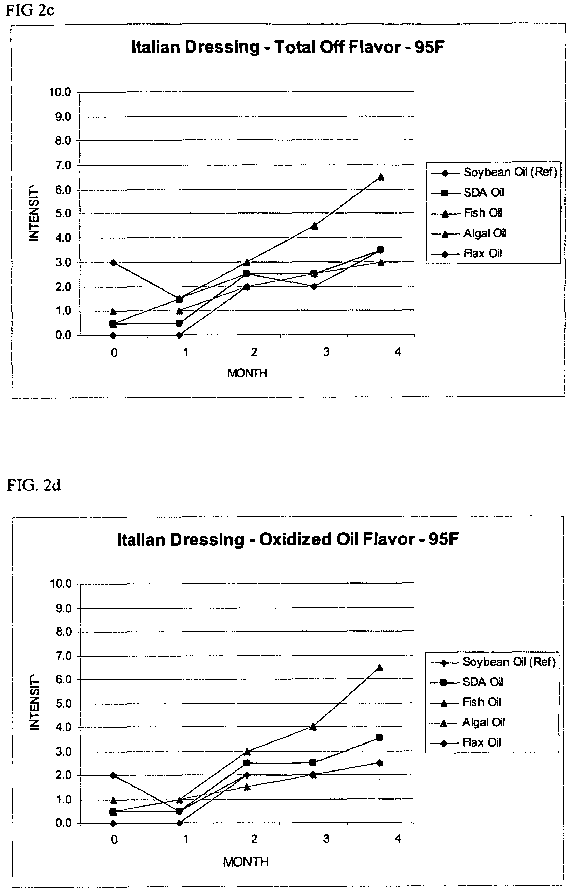

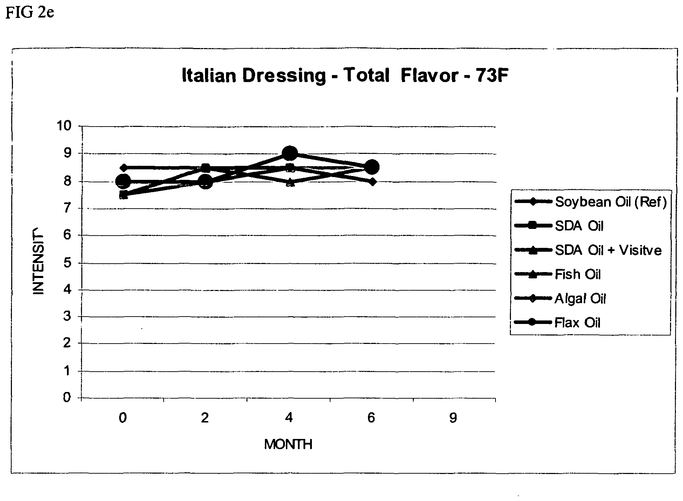

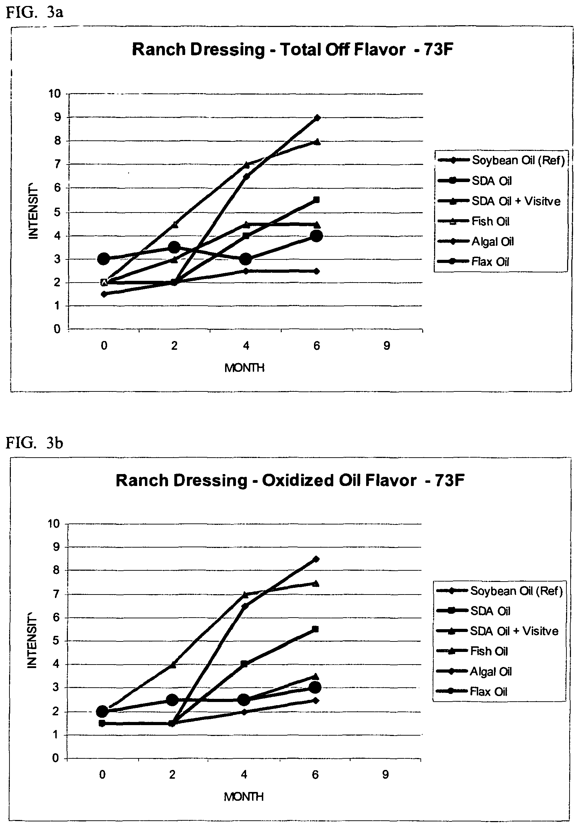

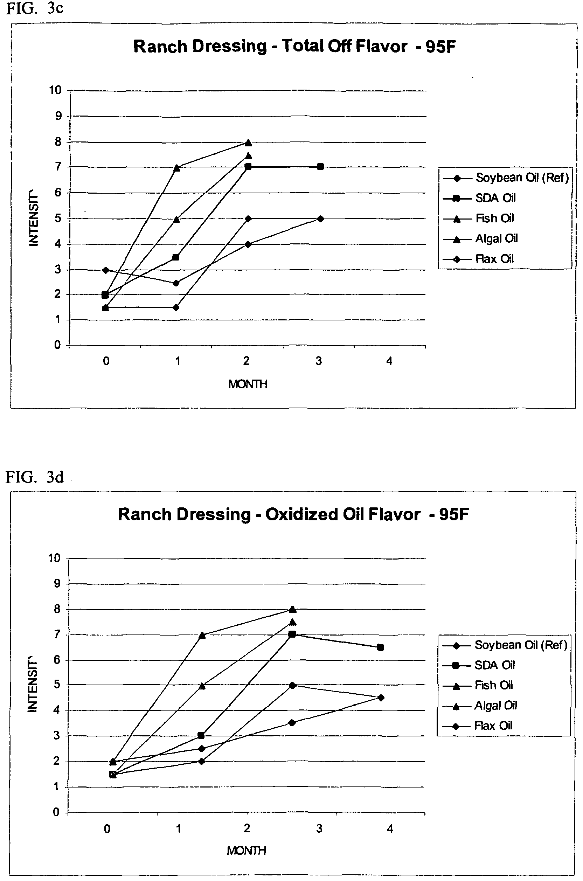

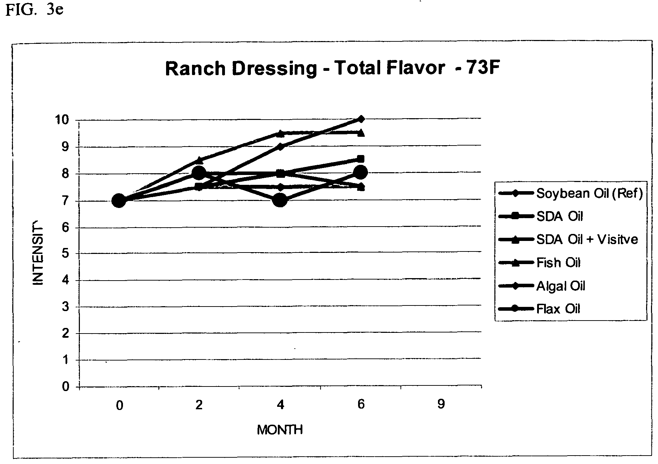

The data for both Italian and Ranch type dressings and charts that demonstrate the characteristics for the evaluation are attached in Tables 1-11 and figures 2 and 3.

### EXAMPLES 2: RANCH SALAD DRESSING

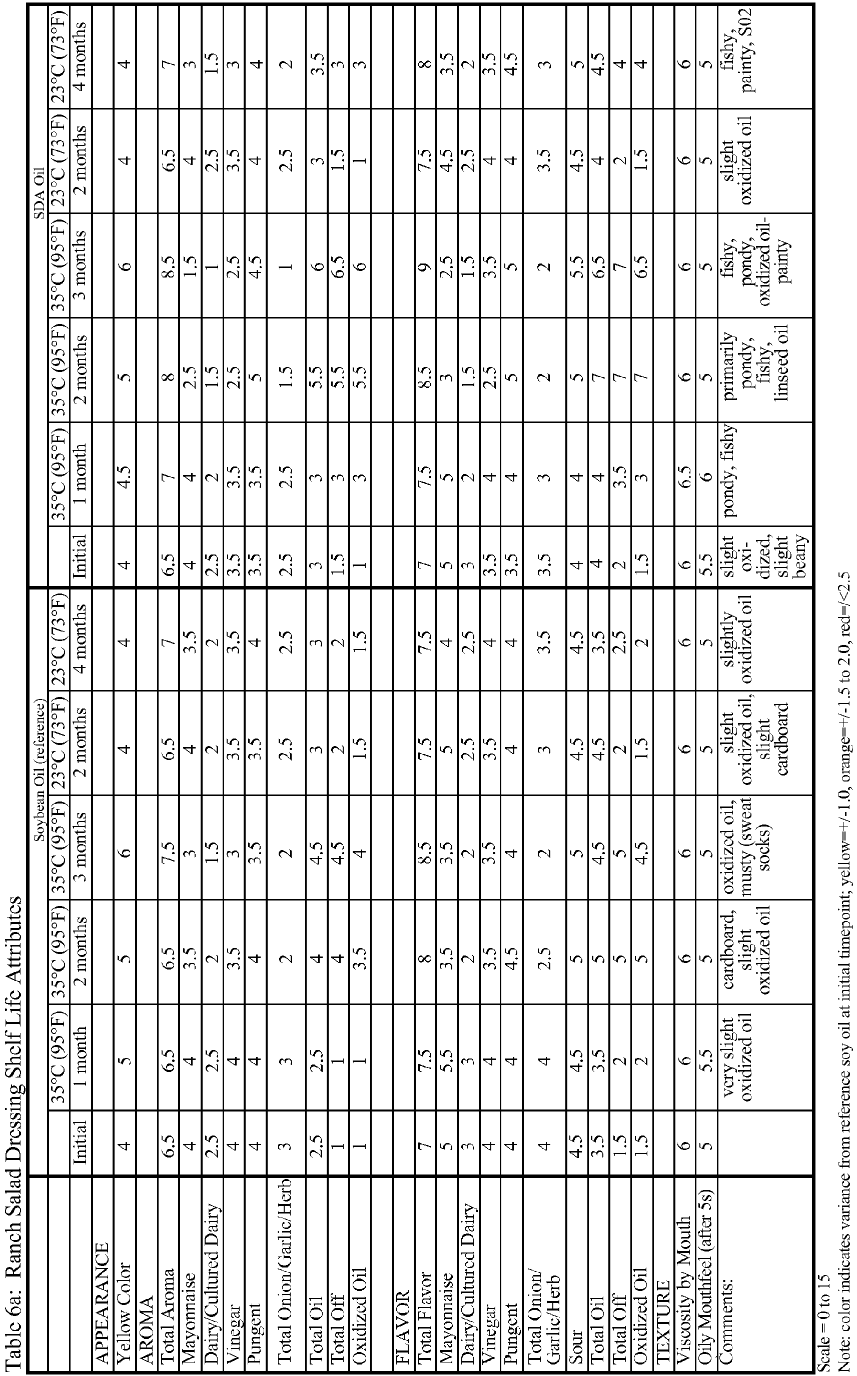

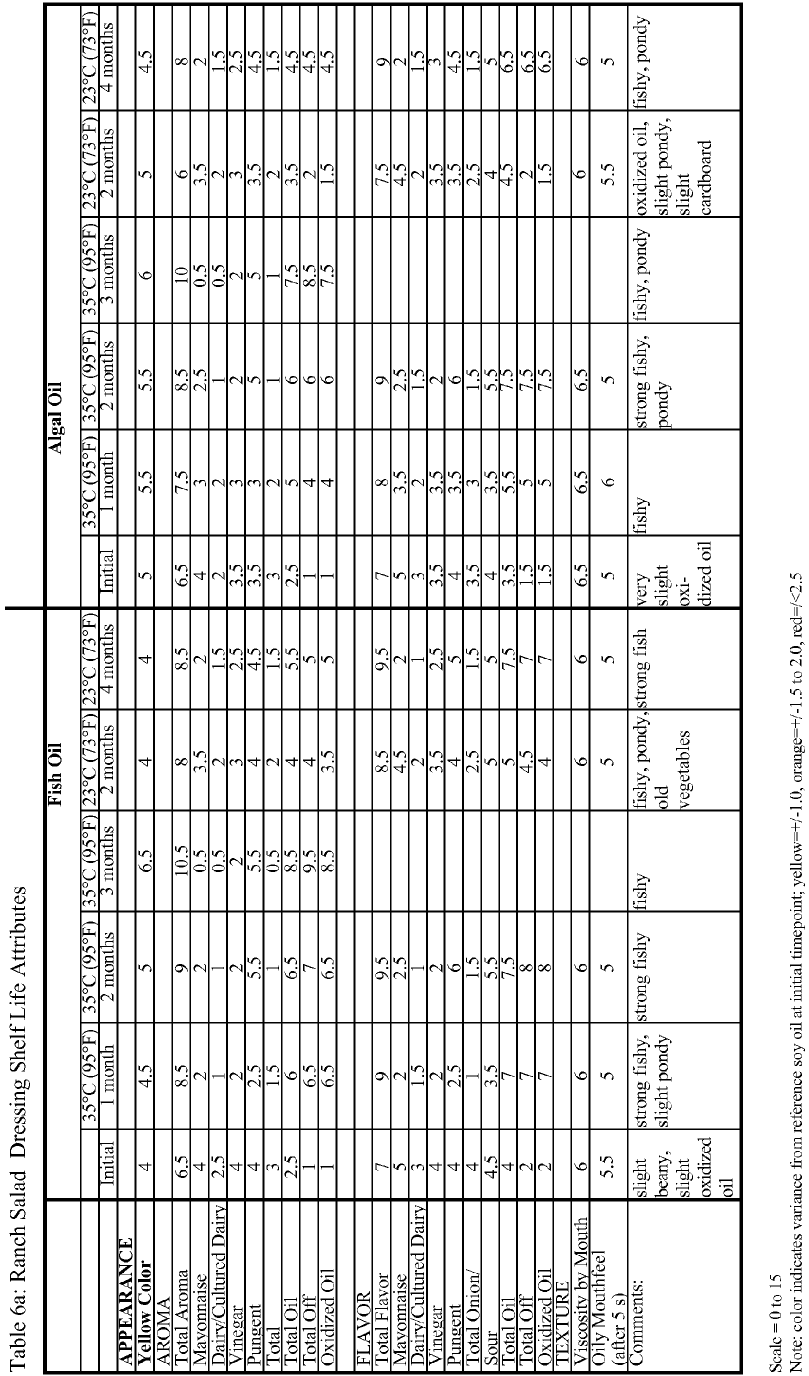

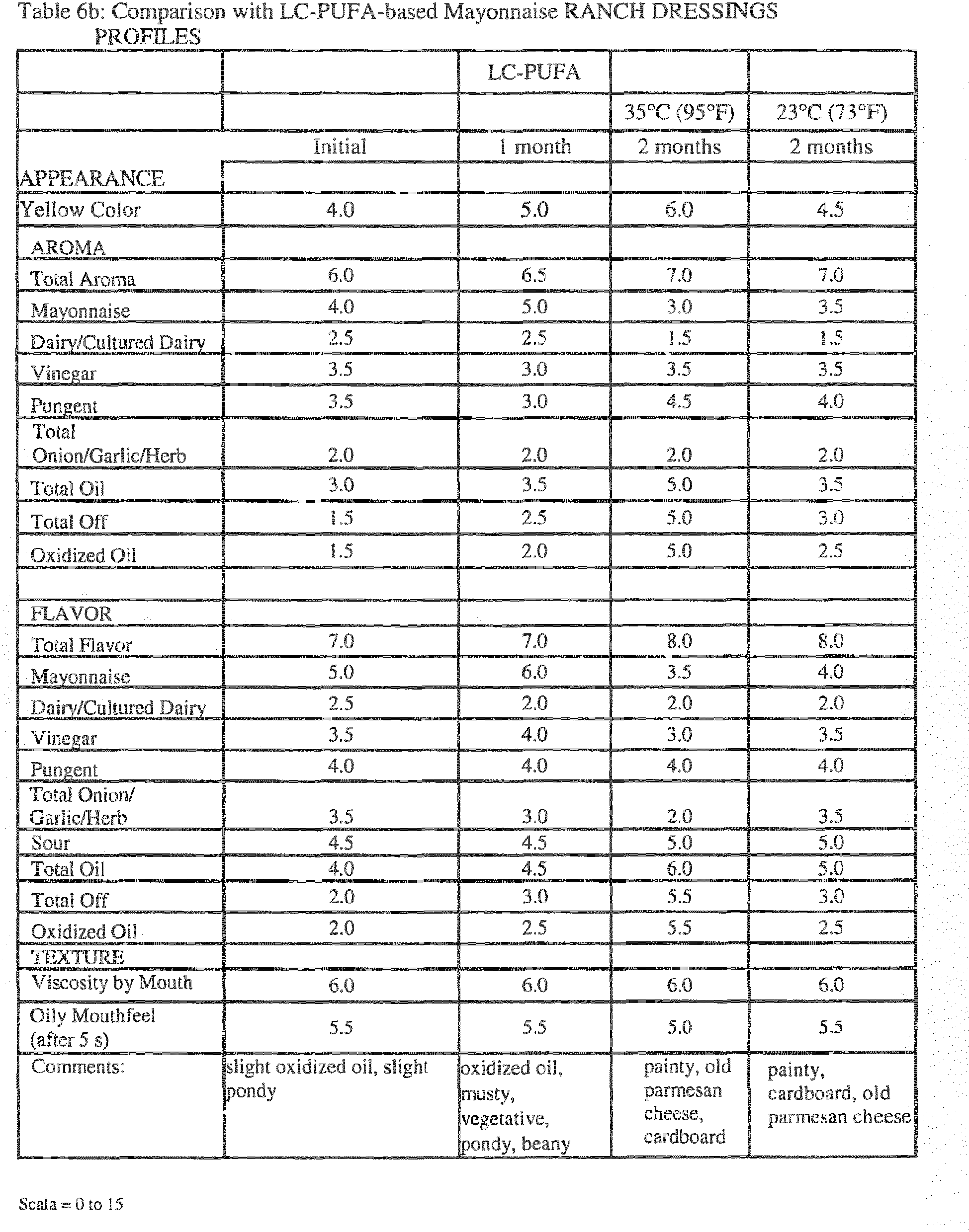

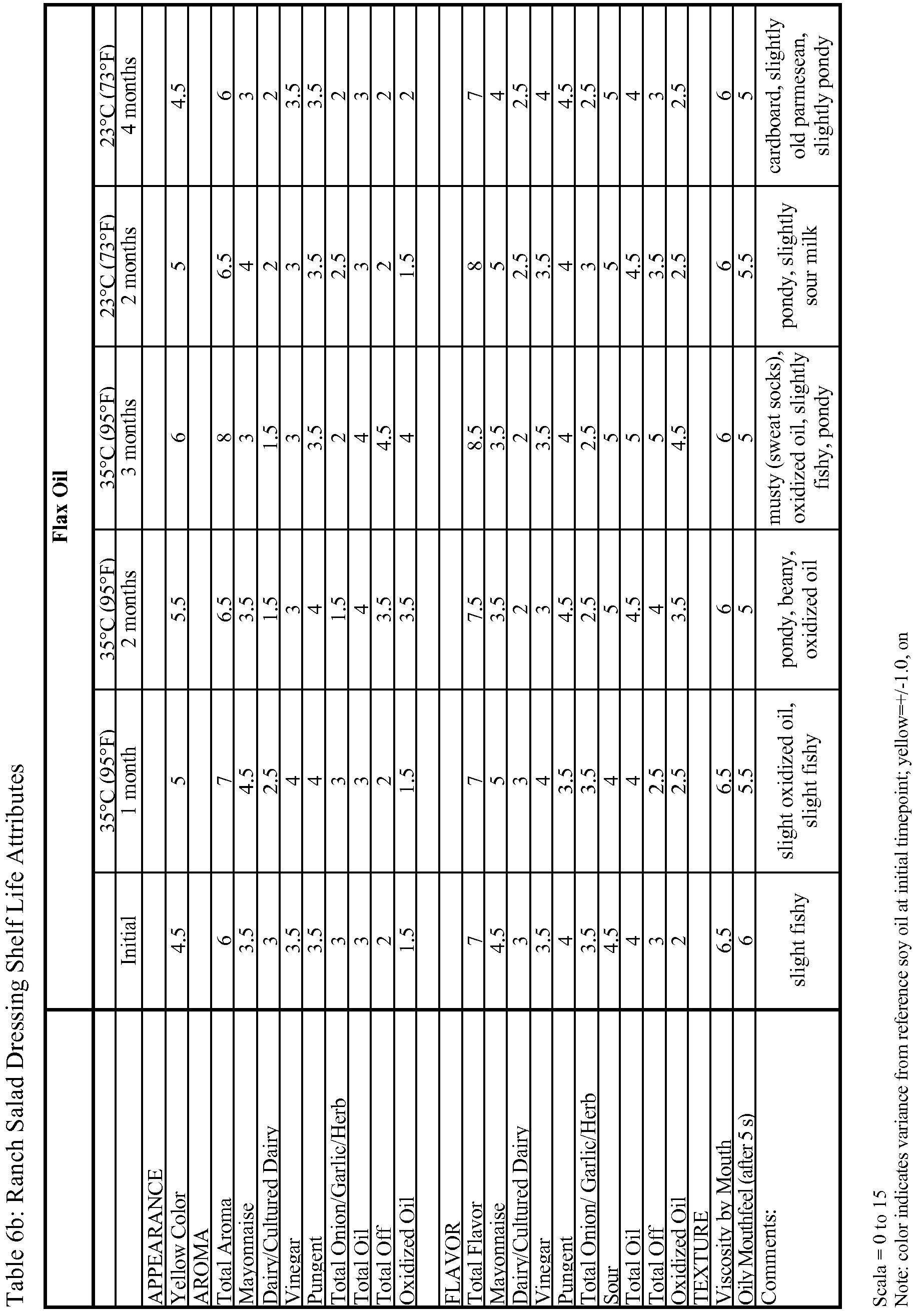

**Table 7a: LC-PUFA SALAD DRESSING FORMULATIONS - RANCH**

| Variant | Control | LC-PUFA | SDA | Fish Oil | Algal Oil | Flax Oil |
|---|---|---|---|---|---|---|
| Formula Number | 50-RA-325-000 | 50-RA-691-000 | 50-RA-326-000 | 50-RA-328-000 | 50-RA-330-000 | 50-RA-327- 000 |
| | | | | | | |
| INGREDIENT | % | | | | | |
| Liquid Soybean Oil | 44.5000 | 33.17 | 33.1700 | 43.0700 | 43.2700 | 42.9700 |
| | | | | | | |
| Omega 3 Oil | | 11.33 | 11.33 | 1.43 | 1.23 | 1.53 |
| Water | 39.3530 | 39.3530 | 39.3530 | 39.3530 | 39.3530 | 39.3530 |
| Egg York, Liquid, 10% Salt | 2.9000 | 2.9000 | 2.9000 | 2.9000 | 2.9000 | 2.9000 |
| Vinegar, White Distilled, 120 g | 2.8500 | 2.8500 | 2.8500 | 2.8500 | 2.8500 | 2.8500 |
| Sugar, White, Fine Granulated | 2.5000 | 2.5000 | 2.5000 | 2.5000 | 2.5000 | 2.5000 |
| Buttermilk Powder, Cultured LOL#20631 | 2.1000 | 2.1000 | 2.1000 | 2.1000 | 2.1000 | 2.1000 |
| Salt, Regular, Non Iodized | 1.7000 | 1.7000 | 1.7000 | 1.7000 | 1.7000 | 1.7000 |
| Flavor, Cultured Buttermilk, Cargill#24521 | 1.5000 | 1.5000 | 1.5000 | 1.5000 | 1.5000 | 1.5000 |
| Garlic, Dehydrated, Granular | 0.4500 | 0.4500 | 0.4500 | 0.4500 | 0.4500 | 0.4500 |
| Onion, Dehydrated, Granular | 0.4400 | 0.4400 | 0.4400 | 0.4400 | 0.4400 | 0.4400 |
| Mustard Flour, Wisconsin Spice SP448 | 0.4000 | 0.4000 | 0.4000 | 0.4000 | 0.4000 | 0.4000 |
| Acid, Phosphoric, 75% | 0.4000 | 0.4000 | 0.4000 | 0.4000 | 0.4000 | 0.4000 |
| Gum, Xanthan, 60 mesh, Regular | 0.2750 | 0.2750 | 0.2750 | 0.2750 | 0.2750 | 0.2750 |
| Preservative, Potassium Sorbate | 02000 | 0.2000 | 0.2000 | 0.2000 | 0.2000 | 0.2000 |
| Monosodium Glutamate (MSG) | 02000 | 0.2000 | 0.2000 | 0.2000 | 0.2000 | 0.2000 |
| Preservative, Sodium Benzoate, Granular | 0.1000 | 0.1000 | 0.1000 | 0.1000 | 0.1000 | 0.1000 |
| Pepper, Black, 30-60 mesh | 0.1000 | 0.1000 | 0.1000 | 0.1000 | 0.1000 | 0.1000 |
| Parsley, Dehydrated, Granular -10 +30 | 0.0250 | 0.0250 | 0.0250 | 0.0250 | 0.0250 | 0.0250 |
| Preservative, EDTA, Calcium Disodium | 0.0070 | 0.0070 | 0.0070 | 0.0070 | 0.0070 | 0.0070 |
| TOTAL | 100.0000 | 100.0000 | 100.0000 | 100.0000 | 100.0000 | 100.0000 |

**Table 7b: Ranch Dressing Production Process**

| |
|---|
| 1. Check that the Mixer is in good working condition, free and clear of any dirt or dust, sealed tight. |
| 2. Set colloid mill at 1.1 cm (0.45"). |
| 3. Set mix tank speed at 45 Hz. |
| 4. Meter water into the mix tank. |
| 5. Add in preservatives (Benzoate, Sorbate, EDTA) into the mix tank. |
| 6. Make gum slurry (Xanthan gum + 700 g soybean oil). |
| 7. Add slurry to dixie tank, allow to mix for 3 min. |
| 8. Increast tank speed to 35 Hz. |
| 9. Add remaining dry ingredients slowly to the mix tank. |
| 10. Add Egg Yolk and Cultured Milk Powder. |
| 11. Increase tank speed to 45 Hz. |
| 12. Slowly add the remaining soybean oil, and if appropriate, the Omega 3 oil. |
| 13. Add slowly, the vinegar and phosphoric acid. |
| 14. All to mix until all ingredients are incorporated and mixed (approx 30 s). |
| 15. Open mix tank valve, and set pump speed to 30 Hz. |

**Table 7c: SHELF LIFE PRODUCTION ANALTYICAUMICRO RESULTS RANCH DRESSING**

| | Control | SDA | Fish Oil | Algal Oil | Flax Oil |
|---|---|---|---|---|---|
| | 50-RA-325-000 | 50-RA-326-000 | 50-RA-328-000 | 50-RA-330-000 | 50-RA-327- 000 |
| pH | 3.80 | 3.79 | 3.79 | 3.79 | 3.80 |
| | | | | | |
| Total Acidity | 0.82 | 0.83 | 0.82 | 0.84 | 0.84 |
| Total Solids | 2.17 | 2.15 | 2.15 | 2.14 | 2.17 |
| Bostwick | 8.3 CM | 8.5 cm | 8.8 cm | 8.5 cm | 8.8 cm |
| Total Plate Count | 30 | 50 | 110 | 30 | 20 |
| Lactics | <10 | <10 | <10 | <10 | <10 |
| Yeast | <10 | <10 | <10 | <10 | <10 |
| Mold | <10 | <10 | <10 | <10 | <10 |

The general approach to the shelf life testing is for 5 trained attribute panelists to taste the dressings and come to consensus regarding the attributes and intensity (on a 15 pt scale - 0 being absent, 15 being extreme) for each dressing. The lists of attributes identified by the panelists are in the attached documents. Additional attributes would be identified as warranted.

### Creamy Ranch Dressing-Initial Time Point

Compared to the reference soybean oil:
The SDA Oil sample did not differ by 1.0 or more for any attribute. Panelists commented that this sample had a slight oxidized, slight beany note.

The LC-PUFA sample was slightly lower in total Onion/Garlic/Herb Aroma. Panelists commented that this sample had a slight oxidized oil, slight pondy note.

The Fish Oil sample did not differ by 1.0 or more for any attribute. Panelists commented that this sample had a slight beany, slight oxidized oil note.

The Algal Oil sample was slightly higher in yellow color. Panelists commented that this sample had a very slight oxidized oil note.

The Flax Oil sample was higher in Total Off Flavor, and slightly Higher in Total Off Aroma and Oily Mouthfeel. Panelists commented that this Sample had a slight fishy flavor

For the current example the tables above provide significant data on flavor and consistency. In the case of Ranch Dressing, because of its more sensitive flavor, the differences between the dressings made with LC-PUFA and the competitive counterparts are more obvious. The tables above represent the data developed for a preferred embodiment of the current invention. Please also see Figures 3a-3h for graphical representation of the data with Ranch Dressing. According to the data provided herein the samples containing LC-PUFA are significantly less off-flavored than those containing the fish and algal oils. Due to pungent flavors and extremely unpleasant odor the fish and algal derived oils were simply removed from the 3 months accelerated evaluation period whereas LC-PUFA was not. Demonstrating improved stability, reduced degradation and consequent enhanced shelf-life.

### EXAMPLE 3: MAYONNAISE

A mayonnaise was prepared and tested with the omega-3 containing oil disclosed herein, the data provided applies for all mayonnaise and spoonable salad dressing variants, produced in a variety of ways (colloid mill and frying mill).

**Table 8a: LC-PUFA - Mayonnaise, Formulation**

| MAYONNAISE SHELF LIFE ATTRIBUTES | | | | | | | | | | |
|---|---|---|---|---|---|---|---|---|---|---|
| n=5 | | | | | | | | | | |
| | Soybean Oil (reference) | | | | | SDA Oil | | | | |
| | | 35°C (95°F) | 35°C (95°F) | 23°C (73°F) | 23°C (73°F) | | 35°C (95°F) | 35°C (95°F) | 23°C (73°F) | 23°C (73°F) |
| | Initial | 1 month | 2 months | 2 months | 4 months | Initial | 1 month | 2 months | 2 months | 4 months |
| APPEARANCE | | | | | | | | | | |
| Color | 4 | 4.5 | 5 | 4 | 4 | 4 | 4.5 | 5 | 4 | 4 |

| AROMA | | | | | | | | | | |
|---|---|---|---|---|---|---|---|---|---|---|
| Total Aroma | 6 | 6.5 | 7 | 6 | 6 | 6 | 7 | 8.5 | 6.5 | 6.5 |
| Eggy Aroma | 3.5 | 3.5 | 3 | 3.5 | 3 | 3.5 | 3.5 | 2 | 3.5 | 2.5 |
| Vinegar Aroma | 3 | 3.5 | 2.5 | 3 | 3 | 3 | 2.5 | 2.5 | 3 | 2.5 |
| Pungent | 4 | 4.5 | 4 | 4 | 4.5 | 3.5 | 4 | 4.5 | 3.5 | 4.5 |
| Total Oil | 1.5 | 2.5 | 3.5 | 2 | 2.5 | 1.5 | 2.5 | 5 | 2 | 3.5 |
| Total Off | 0.5 | 2 | 3.5 | 1.5 | 2.5 | 0.5 | 3 | 6.5 | 2 | 4.5 |
| Oxidized Oil | 0.5 | 2 | 3.5 | 1.5 | 2 | 0.5 | 2.5 | 5 | 2 | 3.5 |

| FLAVOR | | | | | | | | | | |
|---|---|---|---|---|---|---|---|---|---|---|
| Total Flavor | 6.5 | 7 | 7 | 7 | 7 | 6.5 | 8.5 | 9 | 7 | 8 |
| Eggy Flavor | 4 | 4 | 3 | 4 | 3.5 | 4 | 4.5 | 2.5 | 4 | 3 |
| Vinegar Flavor | 2.5 | 3 | 2.5 | 3 | 2.5 | 2.5 | 2.5 | 2.5 | 2.5 | 2.5 |
| Sweet | 3.5 | 3.5 | 3.5 | 3.5 | 3 | 3.5 | 5 | 3.5 | 3 | 3 |
| Sour | 2.5 | 2.5 | 3 | 3 | 3 | 2.5 | 3.5 | 3 | 2.5 | 3 |
| Salty | 3 | 3 | 3 | 3.5 | 3.5 | 3.5 | 3.5 | 3 | 3.5 | 4 |
| Total Oil | 3 | 3.5 | 4 | 3.5 | 3.5 | 3.5 | 4 | 5.5 | 3.5 | 4.5 |
| Total Off | 1.5 | 3 | 4.5 | 2 | 3.5 | 1 | 5 | 6.5 | 2.5 | 5.5 |
| Oxidized Oil | 1.5 | 2.5 | 4 | 2 | 3 | 0.5 | 4 | 5.5 | 2 | 4.5 |

| TEXTURE | | | | | | | | | | |
|---|---|---|---|---|---|---|---|---|---|---|
| Viscosity by Mouth | 8.5 | 8.5 | 8.5 | 8.5 | 8.5 | 8.5 | 8.5 | 8.5 | 8.5 | 9 |
| Oily Mouthfeel (after 5s) | 8.5 | 8.5 | 8.5 | 8.5 | 8.5 | 8.5 | 8.5 | 8.5 | 8.5 | 9 |
| Comments: | | old oil, beany, slightly waxy | painty, cardboard | slightly oxidized, cardboard | reheated oil, slightly beany | | | | slight sulfur, oxidized oil, slightly beany | Slight sulfur, pondy, slightly melted plastic |

**Table 8b Comparison with Fish Oil-based Mayonnaise**

| n=5 | | | | | |
|---|---|---|---|---|---|
| | Fish Oil | | | | |
| | | 35°C (95°F) | 35°C (95°F) | 23°C (73°F) | 23°C (73°F) |
| | Initial | months | 2 months | 2 months | 4 months |
| APPEARANCE | | | | | |
| Color | 4 | 4.5 | 5 | 4 | 4 |

| AROMA | | | | | |
|---|---|---|---|---|---|
| Total Aroma | 6 | 6.5 | 7.5 | 6.5 | 6.5 |
| Eggy Aroma | 3.5 | 3.5 | 3 | 3.5 | 3 |
| Vinegar Aroma | 3 | 3 | 3 | 3 | 3 |
| Pungent | 3.5 | 4 | 4.5 | 4 | 4.5 |
| Total Oil | 1.5 | 2 | 4 | 2.5 | 3 |
| Total Off | 0.5 | 2 | 4.5 | 2 | 3.5 |
| Oxidized Oil | 0.5 | 1.5 | 4 | 2 | 3 |

| FLAVOR | | | | | |
|---|---|---|---|---|---|
| Total Flavor | 6.5 | 7.5 | 8 | 7.5 | 8 |
| Eggy Flavor | 4 | 4 | 2.5 | 4 | 3 |
| Vinegar Flavor | 2.5 | 2.5 | 2.5 | 2.5 | 2.5 |
| Sweet | 3.5 | 3.5 | 3.5 | 3.5 | 3 |
| Sour | 2.5 | 3.5 | 3.5 | 3 | 3 |
| Sally | 3 | 3.5 | 4 | 3.5 | 4 |
| Total Oil | 3 | 3.5 | 5 | 4 | 5 |
| Total Off | 1 | 3 | 6 | 3.5 | 5.5 |
| Oxidized Oil | 0.5 | 2.5 | 5 | 3.5 | 5 |

| TEXTURE | | | | | |
|---|---|---|---|---|---|
| Viscosity by Mouth | 8.5 | 9 | 8.5 | 8.5 | 9 |
| Oily Mouthfeel (after 5 s) | 8.5 | 9 | 8.5 | 8.5 | 9.5 |
| Comments: | | fishy, musty, painty | strong fishy | oxidized oil, painty, old mayo, fish | fishy |

**Table 8c: Comparison with Algal Oil-based Mayonnaise**

| | Algal Oil | | | | |
|---|---|---|---|---|---|
| | | 35°C (95°F) | 35°C (95°F) | 23°C (73°F) | 23°C (73°F) |
| | Initial | 1 month | 2 months | 2 months | 4 months |
| APPEARANCE | | | | | |
| Color | 5.5 | 7 | 6.5 | 6 | 5.5 |

| AROMA | | | | | |
|---|---|---|---|---|---|
| Total Aroma | 6 | 8 | 9 | 7 | 8 |
| Eggy Aroma | 4 | 2.5 | 2 | 3 | 2 |
| Vinegar Aroma | 3 | 3 | 2.5 | 3 | 2 |
| Pungent | 3.5 | 4.5 | 5 | 4 | 5.5 |
| Total Oil | 1.5 | 4 | 6 | 2.5 | 5 |
| Total Off | 0.5 | 4.5 | 6.5 | 2 | 5.5 |
| -Oxidized Oil | 0.5 | 4.5 | 6 | 2 | 5 |

| FLAVOR | | | | | |
|---|---|---|---|---|---|
| Total Flavor | 6.5 | 9 | 9.5 | 8 | 9 |
| Eggy Flavor | 5 | 2.5 | 2 | 3 | 2 |
| Vinegar Flavor | 2.5 | 2.5 | 2 | 2.5 | 1.5 |
| Sweet | 4 | 2.5 | 3.5 | 3 | 3 |
| Sour | 2.5 | 3.5 | 3.5 | 3 | 3.5 |
| Salty | 3.5 | 3.5 | 3.5 | 3.5 | 4 |
| Total Oil | 3 | 6 | 7 | 5 | 6.5 |
| Total Off | 1.5 | 6.5 | 7.5 | 4.5 | 7.5 |
| Oxidized Oil | | 6 | 7 | 4.5 | 6.5 |

| TEXTURE | | | | | |
|---|---|---|---|---|---|
| Viscosity by Mouth | 8.5 | 8.5 | 8.5 | 8.5 | 9 |
| Oily Mouthfeel (after 5 s) | 8.5. | 9 | 8.5 | 8.5 | 8.5 |
| Comments: | | fishy, pondy | Strong fishy | Oxidized oil, painty old mavo. fishy | Fishy, pondy, beany, cardboard |

**Table 8d: Comparison with Flax Oil-based Mayonnaise**

| | Flax Oil | | | | |
|---|---|---|---|---|---|
| | | 35°C (95°F) | 35°C(95°F) | 23°C (73°F) | 23°C (73°F) |
| | Initial | 1 months | 2 months | 2 months | 4 months |
| APPEARANCE | | | | | |
| Color | 4.5 | 5.5 | 5.5 | 5 | 5 |

| AROMA | | | | | |
|---|---|---|---|---|---|
| Total Aroma | 6 | 6.5 | 7.5 | 6.5 | 6.5 |
| Eggy Aroma | 3.5 | 4 | 2 | 3.5 | 2.5 |
| Vinegar Aroma | 3 | 3 | 2.5 | 3.5 | 2.5 |
| Pungent | 3.5 | 4 | 5 | 4.5 | 4 |
| Total Oil | 1.5 | 2.5 | 4.5 | 2 | 3 |
| Total Off | 1.5 | 2 | 51 | 1.5 | 3.5 |
| Oxidized Oil | 1 | 2 | 4.5 | 1.5 | 3 |

| FLAVOR | | | | | |
|---|---|---|---|---|---|
| Total Flavor | 7 | 7 | 8 | 7.5 | 7.5 |
| Eggy Flavor | 3.5 | 4 | 2.5 | 3.5 | 3 |
| Vinegar Flavor | 2.5 | 2.5 | 2 | 3 | 2.5 |
| Sweet | 3 | 3.5 | 3.5 | 3.5 | 3.5 |
| Sour | 2.5 | 3 | 3 | 3 | 3 |
| Salty | 3.5 | 3.5 | 3.5 | | 4 |
| Total Oil | 3 | 3.5 | 5 | 4 | 4.5 |
| Total Off | 3.5 | 2.5 | 5.5 | 3 | 4.5 |
| Oxidized Oil | 3 | 2.5 | 5 | 3 | 4.5 |

| TEXTURE | | | | | |
|---|---|---|---|---|---|
| Viscosity by Mouth | 8.5 | 9 | 8.5 | 8 | 8.5 |
| Oily Mouthfeel | 8.5 | 9 | 8.5 | 8.5 | 8.5 |
| Comments: | | Old oil, reheated oil, beany, waxy | Fishy, cardboard, reheated oil | Fishy, pondy | Strong fishy |

**Table 8e: Comparison with PUFA-based Mayonnaise**

| | Soybean Oil (reference) | SDA Oil | LD-PUFA | Fish Oil | Algal Oil | Flax Oil |
|---|---|---|---|---|---|---|
| | | | | | | |

| | Initial | Initial | Initial | Initial | Initial | Initial |
|---|---|---|---|---|---|---|
| APPEARANCE | | | | | | |
| Color | 4.0 | 4.0 | 3.5 | 4.0 | 5.5 | 4.5 |

| AROMA | | | | | | |
|---|---|---|---|---|---|---|
| Total Aroma | 6.0 | 6.0 | 5.5 | 6.0 | 6.0 | 6.0 |
| Eggy Aroma | 3.5 | 3.5 | 3.0 | 3.5 | 4.0 | 3.5 |
| Vinegar Aroma | 3.0 | 3.0 | 3.0 | 3.0 | 3.0 | 3.0 |
| Pungent | 4.0 | 3.5 | 3.5 | 3.5 | 3.5 | 3.5 |
| Total Oil | 1.5 | 1.5 | 1.5 | 1.5 | 1.5 | 1.5 |
| Total Off | 0.5 | 0.5 | 0.5 | 0.5 | 0.5 | 1.5 |
| Oxidized Oil | 0.5 | 0.5 | 0.5 | 0.5 | 0.5 | 1.0 |

| FLAVOR | | | | | | |
|---|---|---|---|---|---|---|
| Total Flavor | 6.5 | 6.5 | 6.5 | 6.5 | 6.5 | 7.0 |
| Eggy Flavor | 4.0 | 4.0 | 4.0 | 4.0 | 5.0 | 3.5 |
| Vinegar Flavor | 2.5 | 2.5 | 2.5 | 2.5 | 2.5 | 2.5 |
| Sweet | 3.5 | 3.5 | 3.5 | 3.5 | 4. | 3.0 |
| Sour | 2.5 | 2.5 | 2.5 | 2.5 | 2.5 | 2.5 |
| Salty | 3.0 | 3.5 | 3.5 | 3.0 | 3.5 | 3.5 |
| Total Oil | 3.0 | 3.5 | 3.0 | 3.0 | 3.0 | 3.0 |
| Total Off | 1.5 | 1.0 | 1.0 | 1.0 | 1.5 | 3.5 |
| Oxidized Oil | 1.5 | 0.5 | 1.0 | 0.5 | 1.0 | 3.0 |

| TEXTURE | | | | | | |
|---|---|---|---|---|---|---|
| Viscosity by Mouth | 8.5 | 8.5 | 8.5 | 8.5 | 8.5 | 8.5 |
| Oily Mouthfeel (after 5 s) | 8.5 | 8.5 | 9.0 | 8.5 | 8.5 | 8.5 |
| Comments: | Slight cardboard, slight beany | Slight oxidized oil | Very slight oil based paint | Very slight oxidized oil | Slight oxidized oil, slight plastic-like | Fishy, pondy, oxidized oil, reheated oil |
| Scale range = 0 to 15 | | | | | | |
| Note: color indicates variance from Soybean reference; yellow=+/-1.0, orange=+/-1.5 to 2.0, red=)<2.5 | | | | | | |

**Table 9a: LC-PUFA MAYONNAISE FORMULATIONS AND PROCESS**

| Generic Formula | Typical | Range | Control | w/LC-PUFA Oil | w/ Fish Oil | w/Algal oil | w/Flax Oil |
|---|---|---|---|---|---|---|---|
| Control Soybean Oil | 79 | 65-84 | 79.000 | 54.650 | 75.900 | 76.350 | 75.730 |
| LC-PUFA Oil | | | | 24.350 | 3.100 | 2.650 | 3.270 |
| Water | 5.093 | to 100% | 5.093 | 5.093 | 5.093 | 5.093 | 5.093 |
| Egg Yolk (10% Salted) | 7 | 5.0-13.0 | 7.000 | 7.000 | 7.000 | 7.000 | 7.000 |
| White Distilled Vinegar 120 g | 3.5 | 2.0-9.0 | 3.500 | 3.500 | 3.500 | 3.500 | 3.500 |
| Sugar | 3.5 | 1.0-5.0 | 3.500 | 3.500 | 3.500 | 3.500 | 3.500 |
| Salt | 1.4 | 0.5-1.8 | 1.400 | 1.400 | 1.400 | 1.400 | 1.400 |
| Mustard Flour | 0.5 | 0.3-1.0 | 0.500 | 0.500 | 0.500 | 0.500 | 0.500 |
| Calcium Disodium EDTA | 0.007 | 0-0.007 | 0.007 | 0.007 | 0.007 | 0.007 | 0.007 |
| Total | 100 | | 100.00 | 1 00.00 | 100.00 | 100.00 | 100.00 |

| | | | | | | | |
|---|---|---|---|---|---|---|---|
| Notes: Potassium sorbate, lemon juice concentrate, flavorings are optional ingredients. Light and reduced fat versions can be made by reducing fat level and the addition of starch and gum. HFCS and other sweetners may be used in place of sugar. | | | | | | | |

### Public Sources:

21CFR160.10 Standard of Identity for Mayonnasie.
Product Literature: EGGSolutions, American Egg Board.
Product Literature: G. S. Dunn Ltd, Full Egg Mayonnaise.

Process: From G. S. Dunn Ltd Product Literature and known industry practice.
1. Hydrate mustard flour in water for 5 min.
2. Add vinegar, lemon juice (alt. ingredient), salt, sugar to the mixture.
3. Add egg yolk.
   Mix.
4. Add EDTA to the oil.
5. Slowly add the oil to the mix, increasing agitation speed as it is added.
6. Blend and homogenize, utilizing a colloid mill or alternative.

**Table 9b: Mayonnaise Process - Pilot Plant.**

| | |
|---|---|
| 2. | Set the colloid mill at 30. |
| 3. | Add the water first, then mix in the EDTA. |
| 4. | Add the egg yolk, mix for 3 min. |
| 5. | Pre-mix the mustard flour, sugar, and salt. Add the premix slowly until dissolved and evenly dispersed. |
| 6. | Add in the oils mix for 3 min, set Dixie mix tank speed at 35 Hz. |
| 7. | Slowly add in the vinegar. |
| 8. | Mix until all ingredients are dispersed. Shut off Dixie Mixer agitation, allow air to escape. |
| 9. | Start up the Collid Mill. Open mix tank, valve, set pump speed to 30 Hz. |
| 10. | Pack into individual packages. |

The general approach to the shelf life testing is for 5 trained attribute panelists to taste the dressings and come to consensus regarding the attributes and intensity (on a 15 pt scale - 0 being absent, 15 being extreme) for each dressing. The lists of attributes identified by the panelists are in the attached documents. Additional attributes would be identified as warranted.

**Table 9c:**

| | VALUE | SCALE REFERENCE |
|---|---|---|
| APPEARANCE | | |
| Color | 0.0 | White (paper) |
| | 7.5 | Manila Folder |

| AROMA\FLAVOR | | |
|---|---|---|
| Eggy | 8.0/6.0 | Chopped Hard Boiled Eggs |
| Vinegar Aroma | 6.5 | 100% Heinz Distilled Vinegar solution |
| Vinegar Flavor | 4.0 | 2% Heinz Distilled Vinegar solution |
| Total Off | 3.5 | Edamame, raw soybeans |
| Oxidized Dairy/Oil (aroma and flavor) | 4.0 | Canola Oil (opened 9/05) |
| | 5.0 | Wesson Vegetable Oil (opened 11/22/04) |
| | 8.0 | Kraft Parmesan Cheese (2001 expiration date) |
| Sweet | 2.0 | 2.0% Sucrose in Water |
| | 5.0 | 5.0% Sucrose in Water |
| Sour | 2.0 | 0.025% Citric Acid in Water |
| | 5.0 | 0.04% Citric Acid in Water |
| Salty | 2.0 | 0.2% Sodium Chloride in Water |
| | 5.0 | 0.5% Sodium Chloride in Water |

| MOUTHFEEL FACTORS | | |
|---|---|---|
| Pungent (aroma) | 8.0 | 100% Heinz Distilled Vinegar solution |

| TEXTURE | | |
|---|---|---|
| Viscosity by Mouth | 8.0 | 50:50 mix of Lucerne Heavy Cream and Kraft Mayonnaise |
| | 11.0 | Kraft Mayonnaise |
| Oily Mouthfeel | 8.0 | Kraft Mayonnaise |

The following data was developed after initial evaluations. Similar to the Salad Dressings example, the initial flavor of LC-PUFA containing mayonnaise was similar to the control. The flax sample was most different from the others compared.

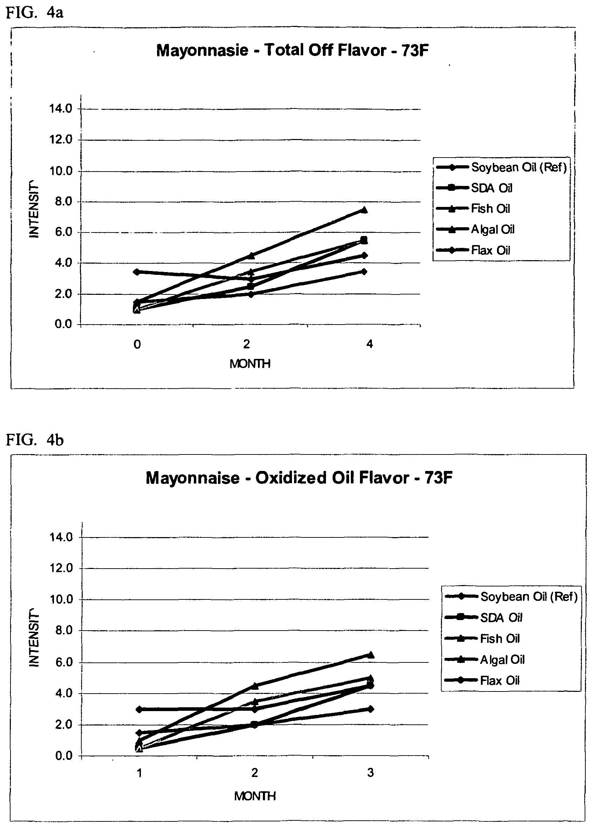

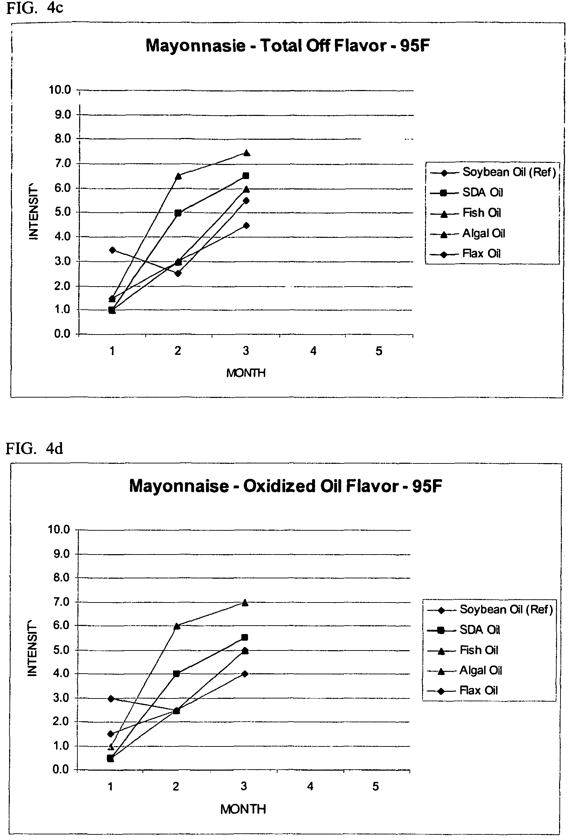

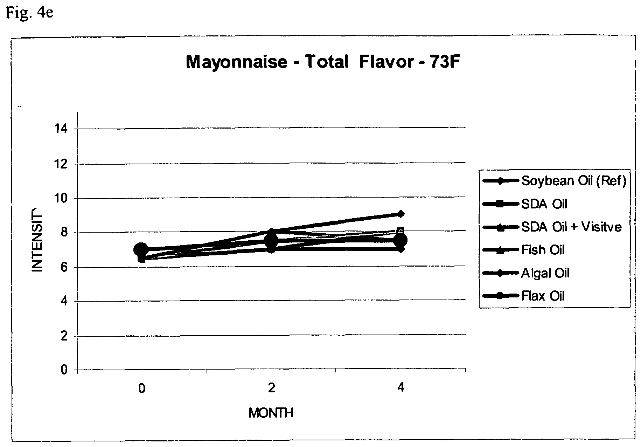

According to the methods disclosed herein, the shelf-life studies two month studies at both room temperature and accelerated storage conditions were completed. All samples in the accelerated temperature study had noticeable off flavor with the algal oil sample containing the highest off notes. LC-PUFA performed better than the other omega-3 containing oil sources. For the room temperature study, Algal oil exhibited much higher levels of off flavors than the LC-PUFA oil of the food/feed products of the invention. See the above data in tables 12-14 and Figures 4a-4e.

### EXAMPLE 4: SOY MILK

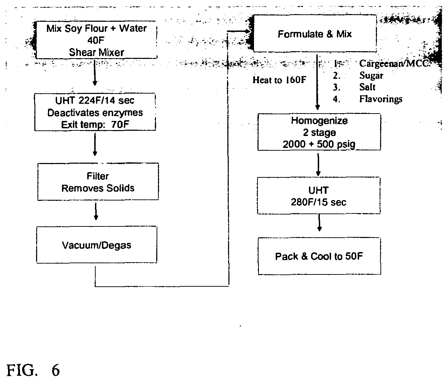

Soymilk can be prepared in two different ways. In the first, LC-PUFA enriched soybeans are de-hulled, flaked and then made into full fatted soy flour. The soymilk is formulated by first dissolving the soy flour into water, mixing, and processing to inactivate the enzymes. The soy base is filtered to remove additional solids and degassed. The remaining ingredients are added, mixed, the product is then homogenized in a two stage homogenizer, then processed through a Ultra High Temperature (UHT) thermal processing unit. The resulting product is packed and refrigerated with a typical shelf life of 12 weeks. Following is a formulation as provided in Table 10, see also FIG. 6 for a process flow diagram.

**Table 10:**

| Vanilla Soymilk | % |
|---|---|
| Water | 88.122 |
| LC-PUFA Enriched Soy Flour | 6.786 |
| Full Fat Soymilk. | 0.600 |
| Sucrose | 3.400 |
| Carageenen | 0.022 |
| Cellulose Gum | 0.350 |
| Salt | 0.040 |
| Calcium Carbonate | 0.350 |
| Natural and Artificial Flavors | 0.330 |
| TOTAL | 100.000 |

The example used can also be applied to different types of homogenization and thermal processing units (direct steam and indirect steam). Different soymilk flavors, including plain, chocolate, apple, orange and berry can be prepared in the same manner.

The resulting product was found to have acceptable flavor and mouth "feel" properties in comparison to soymilk made from flour processed the same way but without the LC-PUFA enhancement of the current invention. According to the data developed in pursuit of the current invention after 9 months shelf life, only slight differences in taste exist between the embodiments of the current invention enhanced with a transgenic LC-PUFA composition versus a control composition with non-transgenic soybean oil containing no Omega-3 fatty acids. This was done for both the soymilk and fruit smoothies. Note these are kept refrigerated and only have a 3 month shelf life in most commercial settings.

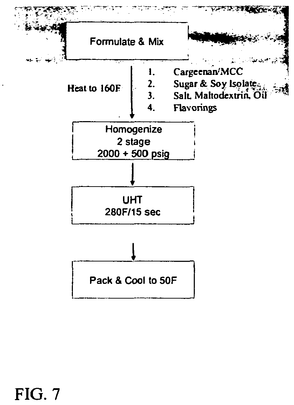

The second approach to this example is to use isolated soy protein, and to add LC-PUFA enriched soy oil to achieve a new product composition. Following is a formulation as provided in Table 11 with a corresponding flow diagram in FIG 7.

**Table 11:**

| Vanilla Soymilk | % |
|---|---|
| Water | 88.058 |
| Sucrose | 3.500 |
| Isolated Soy Protein | 2.700 |
| Maltodextrin | 3.500 |
| 11% LC-PUFA Soybean Oil | 1.500 |
| Carageenan | 0.022 |
| Cellulose gum | 0.350 |
| Salt | 0.040 |
| Natural & Artificial Flavors | 0.330 |
| TOTAL | 100.000 |

The example provided above can also be applied to different types of homogenization and thermal processing units (direct steam and indirect steam.). Different soymilk flavors, including plain, chocolate, apple, orange and berry can be prepared in the same manner. The resulting product was found to have acceptable flavor and mouthfeel properties in comparison to soymilk made with refined, bleached and deodorized soybean oil.

### EXAMPLE 5: FRUIT SMOOTHIES

Fruit smoothies can be developed from soymilk. Other sources of LC-PUFA oil could be used for the development of fruit smoothies as well. Also the processes developed for the production of the fruit smoothies takes into account the unique properties of the LC-PUFA oil for enhancing health and nutrition. Two smoothie type products have been developed, and both products have been determined to have extended shelf life properties. During a process that involves the utilization of ultra high pasteurization, stored refrigerated, with a 12 week shelf life typical of other refrigerated drinks. Although a mixed berry prototype is described herein, other flavors can be developed including strawberry, grape, cranberry, orange, lemon, apple, pineapple, mango, strawberry- banana and any other fruit flavor combination.

In the first approach, soymilk is prepared as described in the first part of Example 4, utilizing LC-PUFA enriched soy flour. Additional ingredients including stabilizers, flavorings and fruit are added prior to homogenization. The following is a formulation used for the product:

**Table 12: MIXED BERRY FRUIT SMOOTHIE - SOY BASED**

| | % |
|---|---|
| Water | 77.774 |
| LC-PUFA Enriched Soy Flour | 6.773 |
| Pectin | 0.300 |
| Cellulose gel/pectin mix | 0.400 |
| Sucrose | 9.300 |
| Citric Acid, anhydrous | 0.450 |
| Potassium Citrate, granular | 0.060 |
| Soy lecithin | 0.060 |
| Salt | 0.070 |
| Frozen Strawberry Puree | 4.000 |
| Frozen Blackberry Puree | 0.500 |
| Red Grape Juice Concentrate | 0.123 |
| Natural Flavor | 0.020 |
| Natural Flavor | 0.060 |
| Natural Berry Flavor | 0.050 |
| Natural and Artificial Mixed Berry Flavor | 0.040 |
| Natural and Artificial Blueberry Flavor | 0.020 |
| Total | 100.000 |

The soybase portion was prepared according to the process described in Example 4. The processing for the remainder of the product is described below:

**Table 13:**

| Preparation Procedures: | |
|---|---|
| 1. | Pre-weigh all dry ingredients |
| 2. | Stabilizer portion: Add prescribed water for stabilizer portion into mixing vessel and begin agitation. |
| 3. | Heat water to 43 to 49°C (110 to 120°F). |
| 4. | Mix the pectin and Avicel with a portion of the dry sugar and add slowly to the water with high shear mixing. Allow 5 minutes for hydration. |
| 5. | Add the citric acid. |
| 6. | Soy milk portion: |
| 7. | Add the potassium citrate, soy lecithin and salt. |
| 8. | Combine the stablilzer portion and soymilk portion into larger, steam jacketed mixing vessel. |
| 9. | Add the purees, color, and flavorings and mix until uniform. |
| 10. | Check pH. Expected pH 4.2 ± 0.2. |
| 11. | Heat to 71°C (160°F) and homogenize d/s 17 + 3.5 mPa (2500+500 psi.) (20.5 mPa (3000 psi) total). |
| 12. | UHT process in the Microthermics unit. Target process is 107°C (224°F) for 19 ss. |
| 13. | Cool in Microthermics cooling sections and fill directly into containers. |
| 14. | Apply closure and place bottles into chilled water bath. Cool to ≤10°C (50°F). |
| 15. | Take count of bottles, apply labels, and refrigerate (PD Warehouse walk-in refrigerator) . |

A second approach developed is where an LC-PUFA enriched oil is added to a formulation containing Isolated Soy Protein. A mixed berry product was developed, but can be extended to additional flavors as described above. Following is the basic formulation used:

**Table 14: MIXED BERRY FRUIT SMOOTHIE - SOY BASED**

| | % |
|---|---|
| Water | 81.077 |
| Pectin | 0.300 |
| Cellulose gel/pectin mix | 0.400 |
| Sucrose | 8.700 |
| Citric Acid, anhydrous | 0.310 |
| 11% LC-PUFA Soybean Oil | 1.500 |
| Isolated Soy Protein | 2.700 |
| Potassium Citrate, granular | 0.060 |
| Soy lecithin | 0.080 |
| Salt | 0.060 |
| Frozen Strawberry Puree | 4.000 |
| Frozen Blackberry Puree | 0.500 |
| Red Grape Juice Concentrate | 0.123 |
| Natural Flavor | 0.020 |
| Natural Flavor | 0.060 |
| Natural Berry Flavor | 0.050 |
| Natural and Artificial Mixed Berry Flavor | 0.040 |
| Natural and Artificial Blueberry Flavor | 0.020 |
| Total | 100.000 |

The product was developed according to the methods disclosed herein and has the following formulation:

**Table 15:**

| Preparation Procedures |
|---|
| 1. Pre-weigh all dry ingredients. |
| 2. Stabilizer portion: Add prescribed water for stabilizer portion into mixing vessel and begin agitation. |
| 3. Heat water to 43 to 49°C (110 to 120°F). |
| 4. Mix the pectin and Avicel with a portion of the dry sugar and add slowly to the water with high shear mixing. Allow 5 min for hydration. |
| 5. Add the citric acid. |
| 6. Soy milk portion: Add the prescribed water for the soymilk portion into a separate mixing vessel and begin agitation. |
| 7. Heat the water to 38 to 43°C (100 to 110°F). |
| 8. Add the soy protein isolate. Mix well to disperse. |
| 9. Add the potassium citrate, soy lecithin, salt and oil. |
| 10. Combine the stablilzer portion and soymilk portion into larger, steam jacketed mixing vessel. |
| 11. Add the frozen strawberry puree, color, and flavorings and mix until uniform. |
| 12. Check pH. Expected pH 4.2 ± 0.2. |

The resulting products from both approaches in this example were typical of a fruit flavored smoothie with a refrigerated shelf life of 12 months.

The data and techniques above demonstrate the production of a mixed berry smoothie from soymilk according to the methods disclosed herein.
The LC-PUFA oil of the food/feed product provides substantial differences relative to other omega-3 containing samples.

### EXAMPLE 6: MARGARINE TYPE SPREADS

**Table 16: 70% Fat Margarine Type Spread**

| | Control | SDA | LC- PUFA | Fish | Algal | Flax |
|---|---|---|---|---|---|---|
| Ingredient | % | % | % | % | % | % |
| Soy Salad Oil | 35.00 | 10.65 | 10.65 | 31.90 | 32.35 | 31.73 |
| Partially Hydrogenated Soy Bean Oil* | 35.00 | 35.00 | 35.00 | 35.00 | 35.00 | 35.00 |
| Omega 3 Oil | | 24.35 | 24.35 | 3.10 | 2.65 | 3.27 |
| | | | | | | |
| Water | 27.60 | 27.60 | 27.60 | 27.60 | 27.60 | 27.60 |
| Salt | 2.00 | 2.00 | 2.00 | 2.00 | 2.00 | 2.00 |
| Lecithin, Soy Based** | 0.14 | 0.14 | 0.14 | 0.14 | 0.14 | 0.14 |
| Sodium Benzoate | 0.09 | 0.09 | 0.09 | 0.09 | 0.09 | 0.09 |
| 52% Plastic Mono & Diglyceride *** | 0.15 | 0.15 | 0.15 | 0.15 | 0.15 | 0.15 |
| Vitamin A / Beta Carotene Blend**** | 0.01 | 0.01 | 0.01 | 0.01 | 0.01 | 0.01 |
| Natural & Artificial Butter Flavor | 0.01 | 0.01 | 0.01 | 0.01 | 0.01 | 0.01 |
| Total | 100.00 | 100.00 | 100.00 | 100.00 | 100.0 | 100.00 |

A typical margarine process, is, the water, salt, sodium benzoate, and butter flavor are mixed as an aqueous phase. Turning to FIG. 7 a milk ingredient, such as whey powder, sodium caseinate or milk powder may be added to the aqueous phase. The oils, lecithin, mono and diglycerides, vitamins, and flavorings are mixed, and combined with the aqueous phase and mixed. The mixed emulsion is passed through a series of scraped surface heat exchangers, pin mixers and resting tubes (A, B and C units respectively) to achieve a desired fill temperature and consistency.

### EXAMPLE 7: COOKIE DOUGH

The LC-PUFA can also be developed into food products including cookies. Below is provided one recipe for such utilization.

**Table 17:**

| Ingredient | % |
|---|---|
| Flour | 49.20 |
| Baker's Sugar | 16.00 |
| Hardened soybean oil (Mpt 36-38°) | 17.40 |
| 20% LC-PUFA Oil | 7.5 |
| Liquid soya oil | 4.1 |
| Salt | 0.80 |
| Water | 5.00 |
| Total | 100.00 |

### Recombinant Plant Production

One method to recombinantly produce a protein of interest a nucleic acid encoding a transgenic protein can be introduced into a host cell. The recombinant host cells can be used to produce the transgenic protein, including a desirable fatty acid such as LC-PUFA that can be secreted or held in the seed, seed pod or other portion of a target plant. A nucleic acid encoding a transgenic protein can be introduced into a host cell, e.g., by homologous recombination. In most cases, a nucleic acid encoding the transgenic protein of interest is incorporated into a recombinant expression vector.

Also described herein are transgenic plants and transformed host cells which comprise, in a 5' to 3' orientation, a promoter operably linked to a heterologous structural nucleic acid sequence. Additional nucleic acid sequences may also be introduced into the plant or host cell along with the promoter and structural nucleic acid sequence. These additional sequences may include 3' transcriptional terminators, 3' polyadenylation signals, other untranslated nucleic acid sequences, transit or targeting sequences, selectable markers, enhancers, and operators.

Preferred nucleic acid sequences, including recombinant vectors, structural nucleic acid sequences, promoters, and other regulatory elements, are described above. The means for preparing such recombinant vectors are well known in the art. For example, methods for making recombinant vectors particularly suited to plant transformation are described in U.S. Pat. Nos. 4,940,835 and 4,757,011.

Typical vectors useful for expression of nucleic acids in cells and higher plants are well known in the art and include vectors derived from the tumor-inducing (Ti) plasmid of *Agrobacterium tumefaciens.* Other recombinant vectors useful for plant transformation, have also been described in the literature.

The transformed host cell may generally be any cell which is compatible with the present invention. The transformed host cell may be prokaryotic, more preferably a bacterial cell, even more preferably an *Agrobacterium*, *Bacillus, Escherichia*, *Pseudomonas* cell, and most preferably is an *Escherichia coli* cell. Alternatively, the transformed host cell is preferably eukaryotic, and more preferably a plant, yeast, or fungal cell. The yeast cell preferably is a *Saccharomyces cerevisiae*, *Schizosaccharomyces pombe,* or *Pichia pastoris*. The plant cell preferably is an alfalfa, apple, banana, barley, bean, broccoli, cabbage, canola, carrot, cassava, celery, citrus, clover, coconut, coffee, corn, cotton, cucumber, garlic, grape, linseed, melon, oat, olive, onion, palm, pea, peanut, pepper, potato, radish, rapeseed (non-canola), rice, rye, sorghum, soybean, spinach, strawberry, sugarbeet, sugarcane, sunflower, tobacco, tomato, or wheat cell. The transformed host cell is more preferably a canola, maize, or soybean cell; and most preferably a soybean cell. The soybean cell is preferably an elite soybean cell line. An "elite line" is any line that has resulted from breeding and selection for superior agronomic performance.

The transgenic plant disclosed herein is preferably an alfalfa, apple, banana, barley, bean, broccoli, cabbage, canola, carrot, cassava, celery, citrus, clover, coconut, coffee, corn, cotton, cucumber, garlic, grape, linseed, melon, oat, olive, onion, palm, pea, peanut, pepper, potato, radish, rapeseed (non-canola), rice, rye, safflower, sorghum, soybean, spinach, strawberry, sugarbeet, sugarcane, sunflower, tobacco, tomato, or wheat plant. The transformed host plant is most preferably a canola, maize, or soybean cell; and of these most preferably a soybean plant.

### Method for Preparing Transgenic Plants

Further disclosed is a method for preparing transgenic plants capable of producing a substantial amount of LC-PUFA comprising, in a 5' to 3' direction, a promoter operably linked to a heterologous structural nucleic acid sequence. The nucleic acid sequence comprising the sequence of LC-PUFA when translated and transcribed into amino acid form. Other structural nucleic acid sequences may also be introduced into the plant along with the promoter and structural nucleic acid sequence. These other structural nucleic acid sequences may include 3' transcriptional terminators, 3' polyadenylation signals, other untranslated nucleic acid sequences, transit or targeting sequences, selectable markers, enhancers, and operators.

The method generally comprises selecting a suitable plant cell, transforming the plant cell with a recombinant vector, obtaining the transformed host cell, and culturing the transformed host cell under conditions effective to produce a plant.

The transgenic plant may generally be any type of plant, preferably is one with agronomic, horticultural, ornamental, economic, or commercial value, and more preferably is an alfalfa, apple, banana, barley, bean, broccoli, cabbage, canola, carrot, castorbean, celery, citrus, clover, coconut, coffee, corn, cotton, cucumber, Douglas fir, Eucalyptus, garlic, grape, Loblolly pine, linseed, melon, oat, olive, onion, palm, parsnip, pea, peanut, pepper, poplar, potato, radish, *Radiata* pine, rapeseed (non-canola), rice, rye, safflower, sorghum, Southern pine, soybean, spinach, strawberry, sugarbeet, sugarcane, sunflower, Sweetgum, tea, tobacco, tomato, turf, or wheat plant. The transformed plant is more preferably a canola, maize, or soybean cell; and most preferably a soybean plant. The soybean plant is preferably an elite soybean plant. An elite plant is any plant from an elite line. Elite lines are described above.

The regeneration, development, and cultivation of plants from transformed plant protoplast or explants is well taught in the art (Gelvin et al., PLANT MOLECULAR BIOLOGY MANUAL, (1990); and, Weissbach and Weissbach, METHODS FOR PLANT MOLECULAR BIOLOGY (1989)). In this method, transformants are generally cultured in the presence of a selective media which selects for the successfully transformed cells and induces the regeneration of the desired plant shoots. These shoots are typically obtained within two to four months.

The shoots are then transferred to an appropriate root-inducing medium containing the selective agent and an antibiotic to prevent bacterial growth. Many of the shoots will develop roots. These are then transplanted to soil or other media to allow the continued development of roots. The method, as outlined, will generally vary depending on the particular plant strain employed.

Preferably, the regenerated transgenic plants are self-pollinated to provide homozygous transgenic plants. Alternatively, pollen obtained from the regenerated transgenic plants may be crossed with non-transgenic plants, preferably inbred lines of economically important species. Conversely, pollen from non-transgenic plants may be used to pollinate the regenerated transgenic plants.

The transgenic plant may pass along the nucleic acid sequence encoding the protein of interest to its progeny. The transgenic plant is preferably homozygous for the nucleic acid encoding the protein of interest protein and transmits that sequence to all its offspring upon as a result of sexual reproduction. Progeny may be grown from seeds produced by the transgenic plant. These additional plants may then be self-pollinated to generate a true breeding line of plants.

The progeny from these plants are evaluated, among other things, for gene expression. The gene expression may be detected by several common methods (e.g., western blotting, immunoprecipitation, and ELISA).

Regulatory sequences include those that direct constitutive expression of a nucleotide sequence in many types of host cells, those that direct expression of the nucleotide sequence only in certain host cells (e.g., tissue-specific regulatory sequences) and those that direct expression in a regulatable manner (e.g., only in the presence of an inducing agent). It will be appreciated by those skilled in the art that the design of the expression vector may depend on such factors as the choice of the host cell to be transformed and the level of expression of transgenic protein desired. The transgenic protein expression vectors can be introduced into host cells to thereby produce transgenic proteins encoded by nucleic acids.

As used herein, the terms "transformation" and "transfection" refer to a variety of art-recognized techniques for introducing foreign nucleic acid (e.g., DNA) into a host cell, including calcium phosphate or calcium chloride co-precipitation, DEAE-dextran-mediated transfection, lipofection, electroporation, microinjection and viral-mediated transfection. Suitable methods for transforming or transfecting host cells can be found in Sambrook et al. (Molecular Cloning: A Laboratory Manual, 2nd Edition, Cold Spring Harbor Laboratory press (1989)), and other laboratory manuals.

One skilled in the art can refer to general reference texts for detailed descriptions of known techniques discussed herein or equivalent techniques. These texts include: Ausubel, et al., CURRENT PROTOCOLS IN MOLECULAR BIOLOGY (eds., John Wiley & Sons, N.Y. (1989)); Birren et al., GENOME ANALYSIS: A LABORATORY MANUAL 1: ANALYZING DNA, (Cold Spring Harbor Press, Cold Spring Harbor, N.Y. (1997)); Clark, PLANT MOLECULAR BIOLOGY: A LABORATORY MANUAL, (Clark, Springer-Verlag, Berlin, (1997)); and, Maliga et al., METHODS IN PLANT MOLECULAR BIOLOGY, (Cold Spring Harbor Press, Cold Spring Harbor, N.Y. (1995)). These texts can, of course, also be referred to in making or using an aspect of the invention. It is understood that any of the agents of the invention can be substantially purified and/or be biologically active and/or recombinant.

### Reduction of Linoleic Acid

It is known that Omega-3 and Omega-6 fatty acids are fatty acids that are required in human nutrition. Omega-6 fatty acids include linoleic acid and its derivatives. These oils are considered essential to human nutrition because these fatty acids must be consumed in the diet because humans cannot manufacture them from other dietary fats or nutrients, and they cannot be stored in the body. Fatty Acids of this sort provide energy and are also components of nerve cells, cellular membranes, and are converted to hormone-like substances known as prostaglandins.

Looking at Fig. 1, linoleic acid is an 18-carbon long polyunsaturated fatty acid containing two double bonds. Its first double bond occurs at the sixth carbon from the omega end, classifying it as an omega-6 oil. As linoleic acid is absorbed and metabolized in the human body, it is converted into a derivative fatty acid, gamma linoleic acid (GLA), which is converted into dihomo-gamma linoleic acid (DGLA) and arachidonic acid (AA). The DGLA and AA are then converted into two types of prostaglandins by adding two carbon molecules and removing hydrogen molecules. There are three families of prostaglandins, PGE1, PGE2, and PGE3. DGLA is converted to PGE1, while AA is converted into PGE2. PGE3 is made by the conversion of omega-3 fatty acids.

In humans the over consumption of omega-6 oils in relation to consumption of omega-3 oils can lead to an overproduction of inflammation-producing prostagladins (PGE2) and a scarcity of anti-inflammatory prostaglandins (PGE1 and PGE2). This in turn can lead to a variety of other health problems. Going further, the daily consumption of omega-6 fatty acids by consumers may be excessive, due to the presence of omega-6 fatty acids in common cooking vegetable oils and processed foods currently on the market. The ratio of omega-6 to omega-3 fatty acid consumption can often reach 20:1 in Western diets. To achieve a more desirable ratio, the increased production of LC-PUFA while reducing the production of LA in a transgenic oilseed plant is envisaged. The resulting oil contains lower levels of LA while providing for the production of significant quantities of LC-PUFA and can be used in a variety of roles in the food industry from cooking oil to food ingredient.

### Raising Tocopherol Levels

Tocopherols are natural antioxidants and essential nutrients in the diet found in plant oils. These antioxidants protect cell membranes and other fat-soluble parts of the body, such as low-density lipoprotein (LDL) cholesterol from damage. It also appears to protect the body against cardiovascular disease and certain forms of cancer and has demonstrated immuno-enhancing effects. Enhancements in the presence of tocopherols in the oil of transgenic seed oil plants will be beneficial to consumers of the oil. Enhanced concentrations of tocopherols will be beneficial as a part of an oil product and may also reduce the oxidation of LC-PUFA

Examples not covered by the scope of the claims are for illustrative purposes.

### Literature Cited:

1. Cohen J.T., et al., A Quantitative Risk-Benefit Analysis Of Changes In Population Fish Consumption. AM J PREV MED. (2005) Nov; 29(4):325-34.
2. Codex Standards For Edible Fats And Oils, in CODEX ALIMENTARIUS COMMISSION: (Supplement 1 to Codex Alimentarius)(Volume XI, Rome, FAO/WHO(1983)).
3. Report of the Fourteenth Session of the Codex Committee an Fats and Oils, London, 27 September - 1 October 1993, CODEX ALIMENTARIUS COMMISSION. (Alinorm 95/17. Rome, FAO/WHO(1993)).
4. DICTIONARY OF FOOD SCIENCE AND TECHNOLOGY, p 141, 151 (Blackwell publ.) (Oxford UK, 2005).
5.Finley, J.W., OMEGA-3 FATTY ACIDS: CHEMISTRY, NUTRITION, AND HEALTH EFFECTS, (ed. John W. Finley) (Publ. American Chemical Society, Wash. DC.)(ACS Symposium, May 2001)(Series Volume: 105-37788).
6. Gebauer S.K., et al., N-3 Fatty Acid Dietary Recommendations And Food Sources To Achieve Essentiality And Cardiovascular Benefits, AM J CLIN NUTR. (2006) Jun; 83(6 Suppl):1526S-1535S.
7. Gelvin et al., PLANT MOLECULAR BIOLOGY MANUAL, (Kluwer Academic Publ. (1990)).
8. Gomez, M.L.M., et al., Sensory Evaluation of Sherry Vinegar: Traditional Compared to Accelerated Aging with Oak Chips, J. FOOD SCIENCE 71(3) S238-S242 (2006).
9. Guichardant M., et al., Stearidonic Acid, an Inhibitor of the 5-Lipoxygenase Pathway, A Comparison With Timnodonic And Dihomogammalinolenic Acid. LIPIDS. (1993) Apr; 28(4):321-24.
10. Gunstone, F.D., and Herslof, B.G. in, LIPID GLOSSARY 2, (Publ. The Oily Press Lipid Library, (2000), 250 pages).
11. Hersleth M., et al., Perception of Bread: A Comparison of Consumers and Trained Assessors, J. FOOD SCIENCE 70(2) S95-101 (2005).
12. James M.J., et al., Metabolism of Stearidonic Acid In Human Subjects: Comparison With The Metabolism of Other N-3 Fatty Acids. AM J CLIN NUTR. 2003 May;77(5):1140-45.
13. Kindle, K., et al., PNAS, USA 87:1228, (1990).
14. Kitamura and Keisuke, Breeding Trials For Improving 77w Food-Processing Quality Of Soybeans, TRENDS FOOD SC1. & TECHNOL. 4:64-67 (1993).
15. La Guardia M., et al., Omega 3 Fatty Acids: Biological Activity And Effects On Human Health, PANMINERVA MED. 2005 Dec;47(4):245-57.
16. Liu, J., et al., Sensory and Chemical Analyses of Oyster Mushrooms (Pleurotus Sajor-Caju) Harvested from Different Substrates, J. FOOD SCIENCE 70(9): S586-S592 (2005).
17. MANUAL ON DESCRIPTIVE ANALYSIS TESTING, FOR SENSORY EVALUATION, (edit. Hootman, R.C., 1992) ASTM Manual Series: MNL 13 pp 1-51 (publ. ASTM).
18. Matta, Z., et al., Consumer and Descriptive Sensory Analysis of Black Walnut Syrup, J. FOOD SCIENCE 70(9): S610-S613 (2005).
19. Morrissey M.T., The Good, The Bad, And The Ugly: Weighing The Risks And Benefits Of Seafood Consumption, NUTR HEALTH. 2006; 18(2):193-7.
20. Myers, R.A. and Worm, B., Rapid World Wide Depletion of Predatory Fish Communities, NATURE 423: 280-83 (2003).
21. O'Brien R.D., FATS AND OILS, FORMULATING AND PROCESSING FOR APPLICATIONS, (publ. CRC Press)(2nd edit. 2003)
22. Omega Pure, FOOD PRODUCT APPLICATIONS, Product Insert (2006).
23. Potrykus, I., ANN. REV. PLANT PHYSIOL. PLANT MOL. BIOLOGY, 42:205, (1991).
24. Rocha-Uribe, A., Physical and Oxidative Stability of Mayonnaise Enriched with Different Levels of n-3 Fatty Acids and stored at Different Temperatures, IF"T ANNUAL MEETING July 12-16 (2004), Las Vegas, USA.
25. Sidel & Stone, Sensory Science: Methodology in, HANDBOOK OF FOOD SCIENCE, TECHNOLOGY AND ENGINEERING VOL. 2, pp. 57-3 through 57-24 (edit. Hui, Y.H., 2005).
26. SOYFOODS COOKBOOK, @ soyfoods.com/recipes. (2006).
27. STANDARD GUIDE FOR SENSORY EVALUATION METHODS TO DETERMINE THE SENSORY SHELF-LIFE OF CONSUMER PRODUCTS, (publ. ASTM Int'l) publication E2454-05; pp. 1-9 (2005).
28. Ursin, V.M., Modification Of Plant Lipids For Human Health:Development Of Functional Land-Based Omega-3 Fatty Acids Symposium: Improving Human Nutrition Through Genomics, Proteomics And Biotechnologies. J. NUTR. 133: 4271-74 (2003).
29. Whelan J. and Rust C., Innovative Dietary Sources of N-3 Fatty Acids, AN-Nu. REV. NUTR. 26: 75-103 (2006).
30. Weissbach and Weissbach, METHODS FOR PLANT MOLECULAR BIOLOGY, (Academic Press, (1989)).
31. Wojciech, K. et al., Possibilities of Fish Oil Application for Food Products Enrichment with Omega-3 PUFA, INT'L J. FooD SCI. NUTR. 50:39-49 (1999).

### Patents and Patent Applications Cited:

### PATENTS:

Abbruzzese, 2002 United States Patent No.# 6,387,883
Akashe et al., 2006, United States Patent No.# 7,037,547
Barclay et al., 1999, United States Patent No.# 5,985,348
Barclay et al., 1997, United States Patent No.# 5,656,319
Barclay et al., 1994, United States Patent No.# 5,340,594
Dartey et al., 2002, United States Patent No.# 6,399,137
Dartey et al., 2000, United States Patent No.# 6,123,978
Knutzon et al., 2002 United States Patent No.# 6,459,018
Schroeder et al., 1990, United States Patent No.# 4,913,921
Wintersdorff et al., 1972, United States Patent No.# 3,676,157

### APPLICATIONS:

Fillatti J., et al., U.S. Patent Application Publication No.2004/0107460A1, June 3, 2004, *Nucleic Acid Constructs and Methods for Producing Altered Seed Oil Compositions*.
Myhre et al., U.S. Patent Application Publication No.2003/0082275A1, May 1, 2003, *Drinkable Omega-3 Preparation and Storage Stabilization.*
Palmer et al., U.S. Patent Application Publication No.2005/0181019A1, Aug 18, 2005, *Nutrition Bar.*
Perlman et al., U.S. Patent Application Publication No.2005/0244564A1, November 3, 2005, *Oxidative Stabilization of Omega-3 Fatty Acids in Low Linoleic Acid-Containing Peanut Butter.*
Shiiba, et al., U.S. Patent Application Publication No.2006/006888A1, March 23, 2004, *Acidic Oil-In- Water Emulsion Compositions*.
Siew, et al., U.S. Patent Application Publication No.2004/0224071A1, November 11, 2004, *Process for Obtaining an Oil Composition and the Oil Composition Obtained Therefrom*.

## Claims

1. Use of a transgenic plant oil comprising stearidonic acid and a soybean oil comprising less than 3 % linolenic acid based on the fatty acid composition of the soybean oil to form a mixture for increasing the shelf-life of a food product.

2. The use of claim 1, wherein the food product further comprises tocopherols.

3. The use of claim 1 or 2, wherein the food product further comprises a) a moisture containing ingredient; and, b) sufficient stabilizer to form an emulsion, such that said product is a stable emulsion.

4. The use of claim 3, wherein said emulsion is of the oil-in-water type and wherein said aqueous phase comprises 10 % to 80 % by weight of said product.

5. The use of claim 4, wherein said aqueous phase comprises water.

6. The use of claim 5, wherein said food product is a salad dressing.

7. The use of claim 3, wherein said moisture containing ingredient is a dairy component.

8. The use of claim 7, wherein said dairy component comprises between 25 % - 80 % of the weight of said product.

9. The use of claim 8, wherein the food product is a yogurt.

10. The use of claim 8, wherein the food product is an ice cream.

11. The use of claim 8, wherein the food product is a margarine.

## Patentansprüche

1. Verwendung eines transgenen Pflanzenöls, das Stearidonsäure umfasst, und eines Sojaöls, das weniger als 3% Linolensäure, bezogen auf die Fettsäurezusammensetzung des Sojaöls, umfasst, zur Bildung eines Gemischs zur Erhöhung der Haltbarkeit eines Lebensmittels.

2. Verwendung gemäß Anspruch 1, wobei das Lebensmittel weiterhin Tocopherole umfasst.

3. Verwendung gemäß Anspruch 1 oder 2, wobei das Lebensmittel weiterhin a) einen feuchtigkeitshaltigen Inhaltsstoff und b) ausreichend Stabilisator zur Bildung einer Emulsion umfasst, so dass das Produkt eine stabile Emulsion ist.

4. Verwendung gemäß Anspruch 3, wobei die Emulsion vom Öl-in-Wasser-Typ ist und wobei die wässrige Phase 10 bis 80 Gew.-% des Produkts umfasst.

5. Verwendung gemäß Anspruch 4, wobei die wässrige Phase Wasser umfasst.

6. Verwendung gemäß Anspruch 5, wobei das Lebensmittel ein Salatdressing ist.

7. Verwendung gemäß Anspruch 3, wobei der feuchtigkeitshaltige Inhaltsstoff eine Milchkomponente ist.

8. Verwendung gemäß Anspruch 7, wobei die Milchkomponente 25% bis 80% des Gewichts des Produkts ausmacht.

9. Verwendung gemäß Anspruch 8, wobei das Lebensmittel ein Joghurt ist.

10. Verwendung gemäß Anspruch 8, wobei das Lebensmittel eine Eiscreme ist.

11. Verwendung gemäß Anspruch 8, wobei das Lebensmittel eine Margarine ist.

## Revendications

1. Utilisation d'une huile de plante transgénique comprenant de l'acide stéaridonique et d'une huile de soja comprenant moins de 3 % d'acide linolénique par rapport à la composition en acides gras de l'huile de soja, pour former un mélange destiné à augmenter la durée de conservation d'un produit alimentaire.

2. Utilisation selon la revendication 1, dans laquelle le produit alimentaire comprend en outre des tocophérols.

3. Utilisation selon la revendication 1 ou 2, dans laquelle le produit alimentaire comprend en outre a) un ingrédient contenant de l'humidité ; et b) suffisamment de stabilisant pour former une émulsion, de façon que ledit produit soit une émulsion stable.

4. Utilisation selon la revendication 3, dans laquelle ladite émulsion est du type huile dans l'eau et dans laquelle ladite phase aqueuse représente 10 % à 80 % en poids dudit produit.

5. Utilisation selon la revendication 4, dans laquelle ladite phase aqueuse comprend de l'eau.

6. Utilisation selon la revendication 5, dans laquelle ledit produit alimentaire est une sauce pour salade.

7. Utilisation selon la revendication 3, dans laquelle ledit ingrédient contenant de l'humidité est un composant laitier.

8. Utilisation selon la revendication 7, dans laquelle ledit composant laitier représente entre 25 % et 80 % en poids dudit produit.

9. Utilisation selon la revendication 8, dans laquelle le produit alimentaire est un yaourt.

10. Utilisation selon la revendication 8, dans laquelle le produit alimentaire est une crème glacée.

11. Utilisation selon la revendication 8, dans laquelle le produit alimentaire est une margarine.
